# EUROPEAN PATENT SPECIFICATION

(11) **EP 0 540 135 B1**
(45) Date of publication and mention of the grant of the patent: **07.06.2000**
(21) Application number: 92302004.4
(22) Date of filing: 10.03.1992
(51) Int. Cl.: C12N 15/45, C12N 7/00, A61K 39/165, C12Q 1/68, C12Q 1/70

(54) **Attenuated measles vaccine virus strain containing specific nucleotide sequence and a method for its absolute identification**
Spezifische Nukleotidsequenz enthaltender Masernvirusvakzinstamm und Verfahren zu seiner absoluten Identifizierung
Souche attenuée vaccinale du virus de la rougeole, contenant une séquence nucléotidique spécifique et procédé pour son identification absolue

(30) Priority: 14.10.1991 JP 29362591
(43) Date of publication of application: 05.05.1993
(73) Proprietor: THE KITASATO INSTITUTE, Minato-ku Tokyo (JP)
(72) Inventor: Sasaki, Keiko, c/o The Kitasato Institute, Tokyo (JP); Mori, Takayuki, c/o The Kitasato Institute, Tokyo (JP); Makino, Satoshi, c/o The Kitasato Institute, Tokyo (JP)
(74) Representative: Woods, Geoffrey Corlett

(56) References cited:
- EP-A- 0 440 219
- THE KITASATO ARCHIVES OF EXPERIMENTAL MEDICINE vol. 47, no. 1/2, June 1974, THE KITASATO INSTITUTE, TOKYO,JAPAN; pages 13 - 21 S. MAKINO ET AL. 'Studies on the modification of the live AIK measles vaccine; II. Development and evaluation of the live AIK-C measles vaccine'
- NUCLEIC ACIDS RESEARCH vol. 15, no. 10, 26 May 1987, IRL PRESS, OXFORD,UK; pages 3987 - 3996 A. SCHMID ET AL. 'A procedure for selective full length cDNA cloning of specific RNA species'
- PCR PROTOCOLS; A GUIDE TO METHODS AND APPLICATIONS 1990, ACADEMIC PRESS , SAN DIEGO, CA, US; pages 378 - 385 D. MACK ET AL. 'Novel viruses'
- JAPAN. J. MICROBIOLOGY vol. 17, no. 1, January 1973, JAPAN pages 75 - 79 S. MAKINO ET AL. 'Field trial with a further attenuated live measles virus vaccine'
- JAPAN. J. MICROBIOLOGY vol. 14, no. 6, November 1970, JAPAN pages 501 - 504 S. MAKINO ET AL. 'Cultivation of measles virus in sheep kidney cells'

## Description

This invention relates the identification of measles virus vaccine strains.

Measles virus, which is the causative virus of measles, which belongs to the Paramyxoviridae family of RNA virus.

The first isolation of measles virus from a patient using a primary culture of human kidney cells was made by J. F. Enders et al. in 1954 ( Enders, J. F. et al. : Proc. Soc. Exp. Biol. Med., 86 : 227 - 286, 1954 ). An attenuated measles vaccine was developed using the isolated Edmonston strain by Enders et al. ( Enders, J. F. et al : New Engl. J. Med., 263 : 153 - 159, 1960 ). The vaccine developed by Enders et al., however, frequently induced adverse effects with pyrogenicity and exanthema. Many strains of attenuated measles virus have been established by means of further attenuation of the Edmonston strain. Among these strains the Schwarz strain established by A. J. F. Schwarz has been commonly used for live measles vaccines.

The inventors of the present invention have isolated four strains of a cold variant derived from an attenuated measles virus of the Edmonston strain supplied by Dr. Enders ( Makino, S. et al., Japan. J. Microbiol., 14 : 501 - 504, 1970 ).

Reduction of immunogenicity, i. e. effectiveness, due to attenuation of measles virus has generally been known. Among the isolated cold variants, a viral strain which adaptively grows at 33°C is a further attenuated measles virus with high immunogenicity having different properties than those generally observed in the conventional measles virus ( Makino, S. et al., Japan. J. Microbiol., 17 ; 75 - 79, 1973 ).

In order to develop the seeds for live measles vaccines from the cold variant, an inventor of the present invention has isolated a virus clone which is a strain adapted with chick embryo cells obtained from specific pathogen free eggs, having same temperature marker, and designated as AIK-C strain ( Sasaki K., Kitasato Arch. Exp. Med., 47 ; 13 - 21, 1974 ).

The pyrogenicity ratio ( ≦ 37.5 °C ) of AIK-C strain live vaccine produced from the seeds of AIK-C strain in measles - sensitive infants is approximately 1/3 - 1/4 that of the Schwarz strain vaccine. The AIK-C strain is more attenuated than the Schwarz strain and possesses a unique characteristic: a high immunogenicity response without lowering immunogenicity ( Makino, S. et al., Kitasato Arch. Exp. Med.,47 ; 13 - 21, 1974).

Encephalitis which seems to be caused by administered live measles vaccine has been observed in from 1 to 3 infants per million of vaccinated infants. No information on such neurological complications has been reported for AIK-C strain live measles vaccine in spite of the administration of this vaccine to ten million people in Japan. ( Hirayama, M. et al., Inf. Dis., 5 ; 495 - 503, 1983, Makino, S., ibid., 5 ; 504 - 505, 1983 ).

The biological properties of the AIK-C strain are unique and different from other strains of measles virus. However, the virus is easily mutated and, even for an attenuated measles virus strain as like the AIK-C strain, a little formation of variant strains can be observed at the growth phase. Accordingly, at the production of measles live vaccine, a quality control check for identity between the seed virus and vaccine virus produced therefrom is most important.

A temperature marker test for AIK-C strain has been applied as a quality control test ( Sasaki, K., Kitasato Arch. Exp. Med., 47 ; 1 - 12, 1974 ). However that is not always an absolute identification method for AIK-C strain.

The fundamental biological properties of a virus depend on its gene, which consists of nucleic acid. The nucleic acid of measles virus consists of single strand negative RNA. Each strain of measles virus has its own nucleic acid consisting of a specific nucleotide sequence.

A complete differential identification method between viral strains is, therefore, to determine the nucleotide sequence of the viral nucleic acid of the strains. The inventors of the present invention has focused on this point and have analysed the nucleotide sequence of nucleic acid relating to measles virus infection, thereby provided an administrative control method for stable AIK-C strain of measles virus without variant and mutation.

Hitherto known identification methods for various measles strain are specific biological response tests However, those are not absolute identification methods. For quality control of the AIK-C strain vaccine, the aforementioned temperature marker test has been applied.

Methods for cloning and sequencing full length RNAs are known, for example Schmid et al., 1987, Nucleic Acids Research, Volume 15, page 3987 to 3996. However it has proved difficult to obtain a definitive sequence for the AIK-C strain because of the high mutation rate of RNA viruses.

However, the inventors of the present invention have now determined the whole nucleotide sequence of the genome of the AIK-C strain virus in order to establish an absolute identification method. Since the entire specific nucleotide sequence consisting of 15,894 bases of the AIK-C strain has been clearly determined, the virus can be identified at the genetic level. The specific nucleotide sequence of 15,894 bases is set out below as Sequence I.D. No. 1. This identification method can thus be an absolute determination method, so that quality control on stable AIK-C strain vaccine can easily be exactly determined. The present invention is thus based on the detection of a specific nucleotide sequence consisting of 15,894 nucleotides in a sequence corresponding to the DNA sequence set out in Sequence I.D. No. 1, in the genomic RNA of a measles vaccine virus strain.

ACCAAACAAA GTTGGGTAAG GATAGTTCAA TCAATGATCA TCTTCTAGTG CACTTAGGAT 60

TCAAGATCCT ATTATCAGGG ACAAGAGCAG GATTAGGGAT ATCCGAG ATG GCC ACA 116

Met Ala Thr

CTT TTA AGG AGC TTA GCA TTG TTC AAA AGA AAC AAG GAC AAA CCA CCC 164

Leu Leu Arg Ser Leu Ala Leu Phe Lys Arg Asn Lys Asp Lys Pro Pro

    5                   10                  15

ATT ACA TCA GGA TCC GGT GGA GCC ATC AGA GGA ATC AAA CAC ATT ATT 212

Ile Thr Ser Gly Ser Gly Gly Ala Ile Arg Gly Ile Lys His Ile Ile

20                  25                  30                  35

ATA GTA CCA ATC CCT GGA GAT TCC TCA ATT ACC ACT CGA TCC AGA CTT 260

Ile Val Pro Ile Pro Gly Asp Ser Ser Ile Thr Thr Arg Ser Arg Leu

                40                  45                  50

CTG GAC CGG TTG GTC AGG TTA ATT GGA AAC CCG GAT GTG AGC GGG CCC 308

Leu Asp Arg Leu Val Arg Leu Ile Gly Asn Pro Asp Val Ser Gly Pro

            55                  60                  65

AAA CTA ACA GGG GCA CTA ATA GGT ATA TTA TCC TTA TTT GTG GAG TCT 356

Lys Leu Thr Gly Ala Leu Ile Gly Ile Leu Ser Leu Phe Val Glu Ser

        70                  75                  80

CCA GGT CAA TTG ATT CAG AGG ATC ACC GAT GAC CCT GAC GTT AGC ATA 404

Pro Gly Gln Leu Ile Gln Arg Ile Thr Asp Asp Pro Asp Val Ser Ile

    85                  90                  95

AGG CTG TTA GAG GTT GTC CAG AGT GAC CAG TCA CAA TCT GGC CTT ACC 452

Arg Leu Leu Glu Val Val Gln Ser Asp Gln Ser Gln Ser Gly Leu Thr

100                 105                 110                 115

TTC GCA TCA AGA GGT ACC AAC ATG GAG GAT GAG GCG GAC AAA TAC TTT 500

Phe Ala Ser Arg Gly Thr Asn Met Glu Asp Glu Ala Asp Lys Tyr Phe

                120                 125                 130

TCA CAT GAT GAT CCA ATT AGT AGT GAT CAA TCC AGG TTC GGA TGG TTC 548
Ser His Asp Asp Pro Ile Ser Ser Asp Gln Ser Arg Phe Gly Trp Phe
            135                 140                 145
GAG AAC AAG GAA ATC TCA GAT ATT GAA GTG CAA GAC CCT GAG GGA TTC 596
Glu Asn Lys Glu Ile Ser Asp Ile Glu Val Gln Asp Pro Glu Gly Phe
        150                 155                 160
AAC ATG ATT CTG GGT ACC ATC CTA GCC CAA ATT TGG GTC TTG CTC GCA 644
Asn Met Ile Leu Gly Thr Ile Leu Ala Gln Ile Trp Val Leu Leu Ala
    165                 170                 175
AAG GCG GTT ACG GCC CCA GAC ACG GCA GCT GAT TCG GAG CTA AGA AGG 692
Lys Ala Val Thr Ala Pro Asp Thr Ala Ala Asp Ser Glu Leu Arg Arg
180                 185                 190                 195
TGG ATA AAG TAC ACC CAA CAA AGA AGG GTA GTT GGT GAA TTT AGA TTG 740
Trp Ile Lys Tyr Thr Gln Gln Arg Arg Val Val Gly Glu Phe Arg Leu
                200                 205                 210
GAG AGA AAA TGG TTG GAT GTG GTG AGG AAC AGG ATT GCC GAG GAC CTC 788
Glu Arg Lys Trp Leu Asp Val Val Arg Asn Arg Ile Ala Glu Asp Leu
            215                 220                 225
TCC TTA CGC CGA TTC ATG GTC GCT CTA ATC CTG GAT ATC AAG AGA ACA 836
Ser Leu Arg Arg Phe Met Val Ala Leu Ile Leu Asp Ile Lys Arg Thr
        230                 235                 240
CCC GGA AAC AAA CCC AGG ATT GCT GAA ATG ATA TGT GAC ATT GAT ACA 884
Pro Gly Asn Lys Pro Arg Ile Ala Glu Met Ile Cys Asp Ile Asp Thr
    245                 250                 255
TAT ATC GTA GAG GCA GGA TTA GCC AGT TTT ATC CTG ACT ATT AAG TTT 932
Tyr Ile Val Glu Ala Gly Leu Ala Ser Phe Ile Leu Thr Ile Lys Phe
260                 265                 270                 275

GGG ATA GAA ACT ATG TAT CCT GCT CTT GGA CTG CAT GAA TTT GCT GGT 980
Gly Ile Glu Thr Met Tyr Pro Ala Leu Gly Leu His Glu Phe Ala Gly
                280                 285                 290
GAG TTA TCC ACA CTT GAG TCC TTG ATG AAC CTT TAC CAG CAA ATG GGG 1028
Glu Leu Ser Thr Leu Glu Ser Leu Met Asn Leu Tyr Gln Gln Met Gly
            295                 300                 305
GAA ACT GCA CCC TAC ATG GTA AAC CTG GAG AAC TCA ATT CAG AAC AAG 1076
Glu Thr Ala Pro Tyr Met Val Asn Leu Glu Asn Ser Ile Gln Asn Lys
        310                 315                 320
TTC AGT GCA GGA TCA TAC CCT CTG CTC TGG AGC TAT GCC ATG GGA GTA 1124
Phe Ser Ala Gly Ser Tyr Pro Leu Leu Trp Ser Tyr Ala Met Gly Val
    325                 330                 335
GGA GTG GAA CTT GAA AAC TCC ATG GGA GGT TTG AAC TTT GGC CGA TCT 1172
Gly Val Glu Leu Glu Asn Ser Met Gly Gly Leu Asn Phe Gly Arg Ser
340                 345                 350                 355
TAC TTT GAT CCA GCA TAT TTT AGA TTA GGG CAA GAG ATG GTA AGG AGG 1220
Tyr Phe Asp Pro Ala Tyr Phe Arg Leu Gly Gln Glu Met Val Arg Arg
                360                 365                 370
TCA GCT GGA AAG GTC AGT TCC ACA TTG GCA TCT GAA CTC GGT ATC ACT 1268
Ser Ala Gly Lys Val Ser Ser Thr Leu Ala Ser Glu Leu Gly Ile Thr
            375                 380                 385
GCC GAG GAT GCA AGG CTT GTT TCA GAG ATT GCA ATG CAT ACT ACT GAG 1316
Ala Glu Asp Ala Arg Leu Val Ser Glu Ile Ala Met His Thr Thr Glu
        390                 395                 400
GAC AAG ATC AGT AGA GCG GTT GGA CCC AGA CAA GCC CAA GTA TCA TTT 1364
Asp Lys Ile Ser Arg Ala Val Gly Pro Arg Gln Ala Gln Val Ser Phe
    405                 410                 415

CTA CAC GGT GAT CAA AGT GAG AAT GAG CTA CCG AGA TTG GGG GGC AAG 1412
Leu His Gly Asp Gln Ser Glu Asn Glu Leu Pro Arg Leu Gly Gly Lys
420                 425                 430                 435
GAA GAT AGG AGG GTC AAA CAG AGT CGA GGA GAA GCC AGG GAG AGC TAC 1460
Glu Asp Arg Arg Val Lys Gln Ser Arg Gly Glu Ala Arg Glu Ser Tyr
                440                 445                 450
AGA GAA ACC GGG CCC AGC AGA GCA AGT GAT GCG AGA GCT GCC CAT CTT 1508
Arg Glu Thr Gly Pro Ser Arg Ala Ser Asp Ala Arg Ala Ala His Leu
            455                 460                 465
CCA ACC GGC ACA CCC CTA GAC ATT GAC ACT GCA TCG GAG TCC AGC CAA 1556
Pro Thr Gly Thr Pro Leu Asp Ile Asp Thr Ala Ser Glu Ser Ser Gln
        470                 475                 480
GAT CCG CAG GAC AGT CGA AGG TCA GCT GAC GCC CTG CTT AGG CTG CAA 1604
Asp Pro Gln Asp Ser Arg Arg Ser Ala Asp Ala Leu Leu Arg Leu Gln
    485                 490                 495
GCC ATG GCA GGA ATC TCG GAA GAA CAA GGC TCA GAC ACG GAC ACC CCT 1652
Ala Met Ala Gly Ile Ser Glu Glu Gln Gly Ser Asp Thr Asp Thr Pro
500                 505                 510                 515
ATA GTG TAC AAT GAC AGA AAT CTT CTA GAC TAGGTGCGAG AGGCCGAGGA 1702
Ile Val Tyr Asn Asp Arg Asn Leu Leu Asp
                520                 525
CCAGAACAAC ATCCGCCTAC CCTCCATCAT TGTTATAAAA AACTTAGGAA CCAGGTCCAC 1762
ACAGCCGCCA GCCCATCAAC CATCCACTCC CACGATTGGA GCCG ATG GCA GAA GAG 1818
Met Ala Glu Glu
CAG GCA CGC CAT GTC AAA AAC GGA CTG GAA TGC ATC CGG GCT CTC AAG 1866
Gln Ala Arg His Val Lys Asn Gly Leu Glu Cys Ile Arg Ala Leu Lys
5 10 15 20

GCC GAG CCC ATC GGC TCA CTG GCC ATC GAG GAA GCT ATG GCA GCA TGG 1914
Ala Glu Pro Ile Gly Ser Leu Ala Ile Glu Glu Ala Met Ala Ala Trp
25 30 35
TCA GAA ATA TCA GAC AAC CCA GGA CAG GAG CGA GCC ACC TGC AGG GAA 1962
Ser Glu Ile Ser Asp Asn Pro Gly Gln Glu Arg Ala Thr Cys Arg Glu
40 45 50
GAG AAG GCA GGC AGT TCG GGT CTC AGC AAA CCA TGC CTC TCA GCA ATT 2010
Glu Lys Ala Gly Ser Ser Gly Leu Ser Lys Pro Cys Leu Ser Ala Ile
55 60 65
GGA TCA ACT GAA GGC GGT GCA CCT CGC ATC CGC GGT CAG GGA CCT GGA 2058
Gly Ser Thr Glu Gly Gly Ala Pro Arg Ile Arg Gly Gln Gly Pro Gly
70 75 80
GAG AGC GAT GAC GAC GCT GAA ACT TTG GGA ATC CCC CCA AGA AAT CTC 2106
Glu Ser Asp Asp Asp Ala Glu Thr Leu Gly Ile Pro Pro Arg Asn Leu
85 90 95 100
CAG GCA TCA AGC ACT GGG TTA CAG TGT TAT TAT GTT TAT GAT CAC AGC 2154
Gln Ala Ser Ser Thr Gly Leu Gln Cys Tyr Tyr Val Tyr Asp His Ser
105 110 115
GGT GAA GCG GTT AAG GGA ATC CAA GAT GCT GAC TCT ATC ATG GTT CAA 2202
Gly Glu Ala Val Lys Gly Ile Gln Asp Ala Asp Ser Ile Met Val Gln
120 125 130
TCA GGC CTT GAT GGT GAT AGC ACC CTA TCA GGA GGA GAC AAT GAA TCT 2250
Ser Gly Leu Asp Gly Asp Ser Thr Leu Ser Gly Gly Asp Asn Glu Ser
135 140 145
GAA AAC AGC GAT GTG GAT ATT GGC GAA CCT GAT ACC GAG GGA TAT GCT 2298
Glu Asn Ser Asp Val Asp Ile Gly Glu Pro Asp Thr Glu Gly Tyr Ala
150 155 160

ATC ACT GAC CGG GGA TCT GCT CCC ATC TCT ATG GGG TTC AGG GCT TCT 2346
Ile Thr Asp Arg Gly Ser Ala Pro Ile Ser Met Gly Phe Arg Ala Ser
165 170 175 180
GAT GTT GAA ACT GCA GAA GGA GGG GAG ATC CAC GAG CTC CTG AGA CTC 2394
Asp Val Glu Thr Ala Glu Gly Gly Glu Ile His Glu Leu Leu Arg Leu
185 190 195
CAA TCC AGA GGC AAC AAC TTT CCG AAG CTT GGG AAA ACT CTC AAT GTT 2442
Gln Ser Arg Gly Asn Asn Phe Pro Lys Leu Gly Lys Thr Leu Asn Val
200 205 210
CCT CCG CCC CCG GAC CCC GGT AGG GCC AGC ACT TCC GGG ACA CCC ATT 2490
Pro Pro Pro Pro Asp Pro Gly Arg Ala Ser Thr Ser Gly Thr Pro Ile
215 220 225
AAA AAG GGC ACA GAG CGC AGA TTA GCC TCA TTT GGA ACG GAG ATC GCG 2538
Lys Lys Gly Thr Glu Arg Arg Leu Ala Ser Phe Gly Thr Glu Ile Ala
230 235 240
TCT TTA TTG ACA GGT GGT GCA ACC CAA TGT GCT CGA AAG TCA CCC TCG 2586
Ser Leu Leu Thr Gly Gly Ala Thr Gln Cys Ala Arg Lys Ser Pro Ser
245 250 255 260
GAA CCA TCA GGG CCA GGT GCA CCT GCG GGG AAT GTC CCC GAG TAT GTG 2634
Glu Pro Ser Gly Pro Gly Ala Pro Ala Gly Asn Val Pro Glu Tyr Val
265 270 275
AGC AAT GCC GCA CTG ATA CAG GAG TGG ACA CCC GAA TCT GGT ACC ACA 2682
Ser Asn Ala Ala Leu Ile Gln Glu Trp Thr Pro Glu Ser Gly Thr Thr
280 285 290
ATC TCC CCG AGA TCC CAG AAT AAT GAA GAA GGG GGA GAC TAT TAT GAT 2730
Ile Ser Pro Arg Ser Gln Asn Asn Glu Glu Gly Gly Asp Tyr Tyr Asp
295 300 305

GAT GAG CTG TTC TCT GAT GTC CAA GAT ATT AAA ACA GCC TTG GCC AAA 2778
Asp Glu Leu Phe Ser Asp Val Gln Asp Ile Lys Thr Ala Leu Ala Lys
310 315 320
ATA CAC GAG GAT AAT CAG AAG ATA ATC TCC AAG CTA GAA TCA CTG CTG 2826
Ile His Glu Asp Asn Gln Lys Ile Ile Ser Lys Leu Glu Ser Leu Leu
325 330 335 340
TTA TTG AAG GGA GAA GTT GAG TCA ATT AAG AAG CAG ATC AAC AGG CAA 2874
Leu Leu Lys Gly Glu Val Glu Ser Ile Lys Lys Gln Ile Asn Arg Gln
345 350 355
AAT ATC AGC ATA TCC ACC CTG GAA GGA CAC CTC TCA AGC ATC ATG ATC 2922
Asn Ile Ser Ile Ser Thr Leu Glu Gly His Leu Ser Ser Ile Met Ile
360 365 370
GCC ATT CCT GGA CTT GGG AAG GAT CCC AAC GAC CCC ACT GCA GAT GTC 2970
Ala Ile Pro Gly Leu Gly Lys Asp Pro Asn Asp Pro Thr Ala Asp Val
375 380 385
GAA ATC AAT CCC GAC TTG AAA CCC ATC ATA GGC AGA GAT TCA GGC CGA 3018
Glu Ile Asn Pro Asp Leu Lys Pro Ile Ile Gly Arg Asp Ser Gly Arg
390 395 400
GCA CTG GCC GAA GTT CTC AAG AAA CCC GTT GCC AGC CGA CAA CTC CAA 3066
Ala Leu Ala Glu Val Leu Lys Lys Pro Val Ala Ser Arg Gln Leu Gln
405 410 415 420
GGA ATG ACA AAT GGA CGG ACC AGT TCC AGA GGA CAG CTG CTG AAG GAA 3114
Gly Met Thr Asn Gly Arg Thr Ser Ser Arg Gly Gln Leu Leu Lys Glu
425 430 435
TTT CAG CCA AAG CCG ATC GGG AAA AAG ATG AGC TCA GCC GTC GGG TTT 3162
Phe Gln Pro Lys Pro Ile Gly Lys Lys Met Ser Ser Ala Val Gly Phe
440 445 450

GTT CCT GAC ACC GGC CCT GCA TCA CGC AGT GTA ATC CGC TCC ATT ATA 3210
Val Pro Asp Thr Gly Pro Ala Ser Arg Ser Val Ile Arg Ser Ile Ile
455 460 465
AAA TCC AGC CGG CTA GAG GAG GAT CGG AAG CGT TAC CTG ATG ACT CTC 3258
Lys Ser Ser Arg Leu Glu Glu Asp Arg Lys Arg Tyr Leu Met Thr Leu
470 475 480
CTT GAT GAT ATC AAA GGA GCC AAT GAT CTT GCC AAG TTC CAC CAG ATG 3306
Leu Asp Asp Ile Lys Gly Ala Asn Asp Leu Ala Lys Phe His Gln Met
485 490 495 500
CTG ATG AAG ATA ATA ATG AAG TAGCTACAGC TCAACTTACC TGCCAACCCC 3357
Leu Met Lys Ile Ile Met Lys
505
ATGCCAGTCG ACCCAACTAG TACAACCTAA ATCCATTATA AAAAACTTAG GAGCAAAGTG 3417
ATTGCCTCCC AAGTTCCACA ATG ACA GAG ATC TAC GAC TTC GAC AAG TCG GCA 3470
Met Thr Glu Ile Tyr Asp Phe Asp Lys Ser Ala
5 10
TGG GAC ATC AAA GGG TCG ATC GCT CCG ATA CAA CCC ACC ACC TAC AGT 3518
Trp Asp Ile Lys Gly Ser Ile Ala Pro Ile Gln Pro Thr Thr Tyr Ser
15 20 25
GAT GGC AGG CTG GTG CCC CAG GTC AGA GTC ATA GAT CCT GGT CTA GGC 3566
Asp Gly Arg Leu Val Pro Gln Val Arg Val Ile Asp Pro Gly Leu Gly
30 35 40
GAC AGG AAG GAT GAA TGC TTT ATG TAC ATG TCT CTG CTG GGG GTT GTT 3614
Asp Arg Lys Asp Glu Cys Phe Met Tyr Met Ser Leu Leu Gly Val Val
45 50 55
GAG GAC AGC GAT CCC CTA GGG CCT CCA ATC GGG CGA GCA TTT GGG TCC 3662
Glu Asp Ser Asp Pro Leu Gly Pro Pro Ile Gly Arg Ala Phe Gly Ser
60 65 70 75

CTG CCC TTA GGT GTT GGC AGA TCC ACA GCA AAG CCC GAA AAA CTC CTC 3710
Leu Pro Leu Gly Val Gly Arg Ser Thr Ala Lys Pro Glu Lys Leu Leu
80 85 90
AAA GAG GCC ACT GAG CTT GAC ATA GTT GTT AGA CGT ACA GCA GGG CTC 3758
Lys Glu Ala Thr Glu Leu Asp Ile Val Val Arg Arg Thr Ala Gly Leu
95 100 105
AAT GAA AAA CTG GTG TTC TAC AAC AAC ACC CCA CTA ACT CTC CTC ACA 3806
Asn Glu Lys Leu Val Phe Tyr Asn Asn Thr Pro Leu Thr Leu Leu Thr
110 115 120
CCT TGG AGA AAG GTC CTA ACA ACA GGG AGT GTC TTC AAC GCA AAC CAA 3854
Pro Trp Arg Lys Val Leu Thr Thr Gly Ser Val Phe Asn Ala Asn Gln
125 130 135
GTG TGC AAT GCG GTT AAT CTG ATA CCG CTC GAT ACC CCG CAG AGG TTC 3902
Val Cys Asn Ala Val Asn Leu Ile Pro Leu Asp Thr Pro Gln Arg Phe
140 145 150 155
CGT GTT GTT TAT ATG AGC ATC ACC CGT CTT TCG GAT AAC GGG TAT TAC 3950
Arg Val Val Tyr Met Ser Ile Thr Arg Leu Ser Asp Asn Gly Tyr Tyr
160 165 170
ACC GTT CCT AGA AGA ATG CTG GAA TTC AGA TCG GTC AAT GCA GTG GCC 3998
Thr Val Pro Arg Arg Met Leu Glu Phe Arg Ser Val Asn Ala Val Ala
175 180 185
TTC AAC CTG CTG GTG ACC CTT AGG ATT GAC AAG GCG ATA GGC CCT GGG 4046
Phe Asn Leu Leu Val Thr Leu Arg Ile Asp Lys Ala Ile Gly Pro Gly
190 195 200
AAG ATC ATC GAC AAT ACA GAG CAA CTT CCT GAG GCA ACA TTT ATG GTC 4094
Lys Ile Ile Asp Asn Thr Glu Gln Leu Pro Glu Ala Thr Phe Met Val
205 210 215

CAC ATC GGG AAC TTC AGG AGA AAG AAG AGT GAA GTC TAC TCT GCC GAT 4142
His Ile Gly Asn Phe Arg Arg Lys Lys Ser Glu Val Tyr Ser Ala Asp
220 225 230 235
TAT TGC AAA ATG AAA ATC GAA AAG ATG GGC CTG GTT TTT GCA CTT GGT 4190
Tyr Cys Lys Met Lys Ile Glu Lys Met Gly Leu Val Phe Ala Leu Gly
240 245 250
GGG ATA GGG GGC ACC AGT CTT CAC ATT AGA AGC ACA GGC AAA ATG AGC 4238
Gly Ile Gly Gly Thr Ser Leu His Ile Arg Ser Thr Gly Lys Met Ser
255 260 265
AAG ACT CTC CAT GCA CAA CTC GGG TTC AAG AAG ACC TTA TGT TAC CCG 4286
Lys Thr Leu His Ala Gln Leu Gly Phe Lys Lys Thr Leu Cys Tyr Pro
270 275 280
CTG ATG GAT ATC AAT GAA GAC CTT AAT CGA TTA CTC TGG AGG AGC AGA 4334
Leu Met Asp Ile Asn Glu Asp Leu Asn Arg Leu Leu Trp Arg Ser Arg
285 290 295
TGC AAG ATA GTA AGA ATC CAG GCA GTT TTG CAG CCA TCA GTT CCT CAA 4382
Cys Lys Ile Val Arg Ile Gln Ala Val Leu Gln Pro Ser Val Pro Gln
300 305 310 315
GAA TTC CGC ATT TAC GAC GAC GTG ATC ATA AAT GAT GAC CAA GGA CTA 4430
Glu Phe Arg Ile Tyr Asp Asp Val Ile Ile Asn Asp Asp Gln Gly Leu
320 325 330
TTC AAA GTT CTG TAGACCGTAG TGCCCAGCAA TGCCCGAAAA CGACCCCCCT 4482
Phe Lys Val Leu
335
CACAATGACA GCCAGAAGGC CCGGACAAAA AAGCCCCCTC CGAAAGACTC CACTGACCAA 4542
GCGAGAGGCC AGCCAGCAGC CGACGGCAAG CACGAACACC AGGCGGCCCC AGCACAGAAC 4602
AGCCCTGATA CAAGGCCACC ACCAGCCACC CCAATCTGCA TCCTCCTCGT GGGACCCCCG 4662
AGGACCAACC CCCAAGGCTG CCCCCGATCC AAACCACCAA CCGCATCCCC ACCACCCCCG 4722
GGAAAGAAAC CCCCAGCAAT TGGAAGGCCC CTCCCCCTCT TCCTCAACAC AAGAACTCCA 4782

CAACCGAACC GCACAAGCGA CCGAGGTGAC CCAACCGCGC GGCATCCGAC TCCCTAGACA 4842

GATCCTCTCT CCCCGGCAAA CTAAACAAAA CTTAGGGCCA AGGAACATAC ACACCCAACA 4902

GAACCCAGAC CCCGGCCCAC GGCGCCGCGC CCCCAACCCC CGACAACCAG AGGGAGCCCC 4962

CAACCAATCC CGCCGGCTCC CCCGGTGCCC ACAGGCAGGG ACACCAACCC CCGAACAGAC 5022

CCAGCACCCA ACCATCGACA ATCCAAGACG GGGGGGCCCC CCCAAAAAAA GGCCCCCAGG 5082

GGCCGACAGC CAGCACCGCG AGGAAGCCCA CCCACCCCAC ACACGACCAC GGCAACCAAA 5142

CCAGAACCCA GACCACCCTG GCCACCAGCT CCCAGACTCG GCCATCACCC CGCAGAAAGG 5202

AAAGGCCACA ACCCGCGCAC CCCAGCCCCG ATCCGGCGGG GAGCCACCCA ACCCGAACCA 5262

GCACCCAAGA GCGATCCCCG AAGGACCCCC GAACCGCAAA GGACATCAGT ATCCCACAGC 5322

CTCTCCAAGT CCCCCGGTCT CCTCCTCTTC TCGAAGGGAC CAAAAGATCA ATCCACCACA 5382

CCCGACGACA CTCAACTCCC CACCCCTAAA GGAGACACCG GGAATCCCAG AATCAAGACT 5442

CATCCAATGT CCATC ATG GGT CTC AAG GTG AAC GTC TCT GCC ATA TTC ATG 5493

Met Gly Leu Lys Val Asn Val Ser Ala Ile Phe Met

5 10

GCA GTA CTG TTA ACT CTC CAA ACA CCC ACC GGT CAA ATC CAT TGG GGC 5541

Ala Val Leu Leu Thr Leu Gln Thr Pro Thr Gly Gln Ile His Trp Gly

15 20 25

AAT CTC TCT AAG ATA GGG GTG GTA GGA ATA GGA AGT GCA AGC TAC AAA 5589

Asn Leu Ser Lys Ile Gly Val Val Gly Ile Gly Ser Ala Ser Tyr Lys

30 35 40

GTT ATG ACT CGT TCC AGC CAT CAA TCA TTA GTC ATA AAA TTA ATG CCC 5637

Val Met Thr Arg Ser Ser His Gln Ser Leu Val Ile Lys Leu Met Pro

45 50 55 60

AAT ATA ACT CTC CTC AAT AAC TGC ACG AGG GTA GAG ATT GCA GAA TAC 5685

Asn Ile Thr Leu Leu Asn Asn Cys Thr Arg Val Glu Ile Ala Glu Tyr

65 70 75

AGG AGA CTA CTG AGA ACA GTT TTG GAA CCA ATT AGA GAT GCA CTT AAT 5733

Arg Arg Leu Leu Arg Thr Val Leu Glu Pro Ile Arg Asp Ala Leu Asn

80 85 90

GCA ATG ACC CAG AAT ATA AGA CCG GTT CAG AGT GTA GCT TCA AGT AGG 5781
Ala Met Thr Gln Asn Ile Arg Pro Val Gln Ser Val Ala Ser Ser Arg
95 100 105
AGA CAC AAG AGA TTT GCG GGA GTA GTC CTG GCA GGT GCG GCC CTA GGC 5829
Arg His Lys Arg Phe Ala Gly Val Val Leu Ala Gly Ala Ala Leu Gly
110 115 120
GTT GCC ACA GCT GCT CAG ATA ACA GCC GGC ATT GCA CTT CAC CAG TCC 5877
Val Ala Thr Ala Ala Gln Ile Thr Ala Gly Ile Ala Leu His Gln Ser
125 130 135 140
ATG CTG AAC TCT CAA GCC ATC GAC AAT CTG AGA GCG AGC CTG GAA ACT 5925
Met Leu Asn Ser Gln Ala Ile Asp Asn Leu Arg Ala Ser Leu Glu Thr
145 150 155
ACT AAT CAG GCA ATT GAG GCA ATC AGA CAA GCA GGG CAG GAG ATG ATA 5973
Thr Asn Gln Ala Ile Glu Ala Ile Arg Gln Ala Gly Gln Glu Met Ile
160 165 170
TTG GCT GTT CAG GGT GTC CAA GAC TAC ATC AAT AAT GAG CTG ATA CCG 6021
Leu Ala Val Gln Gly Val Gln Asp Tyr Ile Asn Asn Glu Leu Ile Pro
175 180 185
TCT ATG AAC CAA CTA TCT TGT GAT TTA ATC GGC CAG AAG CTC GGG CTC 6069
Ser Met Asn Gln Leu Ser Cys Asp Leu Ile Gly Gln Lys Leu Gly Leu
190 195 200
AAA TTG CTC AGA TAC TAT ACA GAA ATC CTG TCA TTA TTT GGC CCC AGC 6117
Lys Leu Leu Arg Tyr Tyr Thr Glu Ile Leu Ser Leu Phe Gly Pro Ser
205 210 215 220
TTA CGG GAC [?]CC ATA TCT GCG GAG ATA TCT ATC CAG GCT TTG AGC TAT 6165
Leu Arg Asp Pro Ile Ser Ala Glu Ile Ser Ile Gln Ala Leu Ser Tyr
225 230 235

GCG CTT GGA GGA GAC ATC AAT AAG GTG TTA GAA AAG CTC GGA TAC AGT 6213
Ala Leu Gly Gly Asp Ile Asn Lys Val Leu Glu Lys Leu Gly Tyr Ser
240 245 250
GGA GGT GAT TTA CTG GGC ATC TTA GAG AGC AGA GGA ATA AAG GCC CGG 6261
Gly Gly Asp Leu Leu Gly Ile Leu Glu Ser Arg Gly Ile Lys Ala Arg
255 260 265
ATA ACT CAC GTC GAC ACA GAG TCC TAC TTA ATT GTC CTC AGT ATA GCC 6309
Ile Thr His Val Asp Thr Glu Ser Tyr Leu Ile Val Leu Ser Ile Ala
270 275 280
TAT CCG ACG CTG TCC GAG ATT AAG GGG GTG ATT GTC CAC CGG CTA GAG 6357
Tyr Pro Thr Leu Ser Glu Ile Lys Gly Val Ile Val His Arg Leu Glu
285 290 295 300
GGG GTC TCG TAC AAC ATA GGC TCT CAA GAG TGG TAT ACC ACT GTG CCC 6405
Gly Val Ser Tyr Asn Ile Gly Ser Gln Glu Trp Tyr Thr Thr Val Pro
305 310 315
AAG TAT GTT GCA ACC CAA GGG TAC CTT ATC TCG AAT TTT GAT GAG TCA 6453
Lys Tyr Val Ala Thr Gln Gly Tyr Leu Ile Ser Asn Phe Asp Glu Ser
320 325 330
TCG TGT ACT TTC ATG CCA GAG GGG ACT GTG TGC AGC CAA AAT GCC TTG 6501
Ser Cys Thr Phe Met Pro Glu Gly Thr Val Cys Ser Gln Asn Ala Leu
335 340 345
TAC CCG ATG AGT CCT CTG CTC CAA GAA TGC CTC CGG GGG TCC ACC AAG 6549
Tyr Pro Met Ser Pro Leu Leu Gln Glu Cys Leu Arg Gly Ser Thr Lys
350 355 360
TCC TGT GCT CGT ACA CTC GTA TCC GGG TCT TTT GGG AAC CGG TTC ATT 6597
Ser Cys Ala Arg Thr Leu Val Ser Gly Ser Phe Gly Asn Arg Phe Ile
365 370 375 380

TTA TCA CAA GGG AAC CTA ATA GCC AAT TGT GCA TCA ATC CTT TGC AAG 6645
Leu Ser Gln Gly Asn Leu Ile Ala Asn Cys Ala Ser Ile Leu Cys Lys
385 390 395
TGT TAC ACA ACA GGA ACG ATC ATT AAT CAA GAC CCT GAC AAG ATC CTA 6693
Cys Tyr Thr Thr Gly Thr Ile Ile Asn Gln Asp Pro Asp Lys Ile Leu
400 405 410
ACA TAC ATT GCT GCC GAT CAC TGC CCG GTA GTC GAG GTG AAC GGC GTG 6741
Thr Tyr Ile Ala Ala Asp His Cys Pro Val Val Glu Val Asn Gly Val
415 420 425
ACC ATC CAA GTC GGG AGC AGG AGG TAT CCA GAC GCT GTG TAC TTG CAC 6789
Thr Ile Gln Val Gly Ser Arg Arg Tyr Pro Asp Ala Val Tyr Leu His
430 435 440
AGA ATT GAC CTC GGT CCT CCC ATA TTA TTG GAG AGG TTG GAC GTA GGG 6837
Arg Ile Asp Leu Gly Pro Pro Ile Leu Leu Glu Arg Leu Asp Val Gly
445 450 455 460
ACA AAT CTG GGG AAT GCA ATT GCT AAG TTG GAG GAT GCC AAG GAA TTG 6885
Thr Asn Leu Gly Asn Ala Ile Ala Lys Leu Glu Asp Ala Lys Glu Leu
465 470 475
TTG GAG TCA TCG GAC CAG ATA TTG AGG AGT ATG AAA GGT TTA TCG AGC 6933
Leu Glu Ser Ser Asp Gln Ile Leu Arg Ser Met Lys Gly Leu Ser Ser
480 485 490
ACT TGC ATA GTC TAC ATC CTG ATT GCA GTG TGT CTT GGA GGG TTG ATA 6981
Thr Cys Ile Val Tyr Ile Leu Ile Ala Val Cys Leu Gly Gly Leu Ile
495 500 505
GGG ATC CCC GCT TTA ATA TGT TGC TGC AGG GGG CGT TGT AAC AAA AAG 7029
Gly Ile Pro Ala Leu Ile Cys Cys Cys Arg Gly Arg Cys Asn Lys Lys
510 515 520

GGA GAA CAA GTT GGT ATG TCA AGA CCA GGC CTA AAG CCT GAT CTT ACG 7077
Gly Glu Gln Val Gly Met Ser Arg Pro Gly Leu Lys Pro Asp Leu Thr
525 530 535 540
GGA ACA TCA AAA TCC TAT GTA AGG TCG CTC TGATCCTCTA CAACTCTTGA 7127
Gly Thr Ser Lys Ser Tyr Val Arg Ser Leu
545 550
AACACAAATG TCCCACAAGT CTCCTCTTCG TCATCAAGCA ACCACCGCAC CCAGCATCAA 7187
GCCCACCTGA AATTATCTCC GGCTTCCCTC TGGCCGAACA ATATCGGTAG TTAATTAAAA 7247
CTTAGGGTGC AAGATCATCC ACA ATG TCA CCA CAA CGA GAC CGG ATA AAT GCC 7300
Met Set Pro Gln Arg Asp Arg Ile Asn Ala
5 10
TTC TAC AAA GAT AAC CCC CAT CCC AAG GGA AGT AGG ATA GTC ATT AAC 7348
Phe Tyr Lys Asp Asn Pro His Pro Lys Gly Ser Arg Ile Val Ile Asn
15 20 25
AGA GAA CAT CTT ATG ATT GAT AGA CCT TAT GTT TTG CTG GCT GTT CTG 7396
Arg Glu His Leu Met Ile Asp Arg Pro Tyr Val Leu Leu Ala Val Leu
30 35 40
TTT GTC ATG TTT CTG AGC TTG ATC GGG TTG CTA GCC ATT GCA GGC ATT 7444
Phe Val Met Phe Leu Ser Leu Ile Gly Leu Leu Ala Ile Ala Gly Ile
45 50 55
AGA CTT CAT CGG GCA GCC ATC TAC ACC GCA GAG ATC CAT AAA AGC CTC 7492
Arg Leu His Arg Ala Ala Ile Tyr Thr Ala Glu Ile His Lys Ser Leu
60 65 70
AGC ACC AAT CTA GAT GTA ACT AAC TCA ATC GAG CAT CAG GTC AAG GAC 7540
Ser Thr Asn Leu Asp Val Thr Asn Ser Ile Glu His Gln Val Lys Asp
75 80 85 90
GTG CTG ACA CCA CTC TTC AAA ATC ATC GGT GAT GAA GTG GGC CTG AGG 7588
Val Leu Thr Pro Leu Phe Lys Ile Ile Gly Asp Glu Val Gly Leu Arg
95 100 105

ACA CCT CAG AGA TTC ACT GAC CTA GTG AAA TTC ATC TCT GAC AAG ATT 7636
Thr Pro Gln Arg Phe Thr Asp Leu Val Lys Phe Ile Ser Asp Lys Ile
110 115 120
AAA TTC CTT AAT CCG GAT AGG GAG TAC GAC TTC AGA GAT CTC ACT TGG 7684
Lys Phe Leu Asn Pro Asp Arg Glu Tyr Asp Phe Arg Asp Leu Thr Trp
125 130 135
TGT ATG AAC CCG CCA GAG AGA ATC AAA TTG GAT TAT GAT CAA TAC TGT 7732
Cys Ile Asn Pro Pro Glu Arg Ile Lys Leu Asp Tyr Asp Gln Tyr Cys
140 145 150
GCA GAT GTG GCT GCT GAA GAG CTC ATG AAT GCA TTG GTG AAC TCA ACT 7780
Ala Asp Val Ala Ala Glu Glu Leu Met Asn Ala Leu Val Asn Ser Thr
155 160 165 170
CTA CTG GAG ACC AGA ACA ACC AAT CAG TTC CTA GCT GTC TCA AAG GGA 7828
Leu Leu Glu Thr Arg Thr Thr Asn Gln Phe Leu Ala Val Ser Lys Gly
175 180 185
AAC TGC TCA GGG CCC ACT ACA ATC AGA GGT CAA TTC TCA AAC ATG TCG 7876
Asn Cys Ser Gly Pro Thr Thr Ile Arg Gly Gln Phe Ser Asn Met Ser
190 195 200
CTG TCC CTG TTA GAC TTG TAT TTA GGT CGA GGT TAC AAT GTG TCA TCT 7924
Leu Ser Leu Leu Asp Leu Tyr Leu Gly Arg Gly Tyr Asn Val Ser Ser
205 210 215
ATA GTC ACT ATG ACA TCC CAG GGA ATG TAT GGG GGA ACT TAC CTA GTG 7972
Ile Val Thr Met Thr Ser Gln Gly Met Tyr Gly Gly Thr Tyr Leu Val
220 225 230
GAA AAG CCT AAT CTG AGC AGC AAA AGG TCA GAG TTG TCA CAA CTG AGC 8020
Glu Lys Pro Asn Leu Ser Ser Lys Arg Ser Glu Leu Ser Gln Leu Ser
235 240 245 250

ATG TAC CGA GTG TTT GAA GTA GGT GTT ATC AGA AAT CCG GGT TTG GGG 8068
Met Tyr Arg Val Phe Glu Val Gly Val Ile Arg Asn Pro Gly Leu Gly
255 260 265
GCT CCG GTG TTC CAT ATG ACA AAC TAT CTT GAG CAA CCA GTC AGT AAT 8116
Ala Pro Val Phe His Met Thr Asn Tyr Leu Glu Gln Pro Val Ser Asn
270 275 280
GAT CTC AGC AAC TGT ATG GTG GCT TTG GGG GAG CTC AAA CTC GCA GCC 8164
Asp Leu Ser Asn Cys Met Val Ala Leu Gly Glu Leu Lys Leu Ala Ala
285 290 295
CTT TGT CAC CGG GAA GAT TCT ATC ACA ATT CCC TAT CAG GGA TCA GGG 8212
Leu Cys His Arg Glu Asp Ser Ile Thr Ile Pro Tyr Gln Gly Ser Gly
300 305 310
AAA GGT GTC AGC TTC CAG CTC GTC AAG CTA GGT GTC TGG AAA TCC CCA 8260
Lys Gly Val Ser Phe Gln Leu Val Lys Leu Gly Val Trp Lys Ser Pro
315 320 325 330
ACC GAC ATG CAA TCC TGG GTC ACC TTA TCA ACG GAT GAT CCA GTG ATA 8308
Thr Asp Met Gln Ser Trp Val Thr Leu Ser Thr Asp Asp Pro Val Ile
335 340 345
GAC AGG CTT TAC CTC TCA TCT CAC AGA GGT GTT ATC GCT GAC AAT CAA 8356
Asp Arg Leu Tyr Leu Ser Ser His Arg Gly Val Ile Ala Asp Asn Gln
350 355 360
GCA AAA TGG GCT GTC CCG ACA ACA CGA ACA GAT GAC AAG TTG CGA ATG 8404
Ala Lys Trp Ala Val Pro Thr Thr Arg Thr Asp Asp Lys Leu Arg Met
365 370 375
GAG ACA TGC TTC CAA CAG GCG TGT AAG GGT AAA ATC CAA GCA CTC TGC 8452
Glu Thr Cys Phe Gln Gln Ala Cys Lys Gly Lys Ile Gln Ala Leu Cys
380 385 390

GAG AAT CCC GAG TGG GCA CCA TTG AAG GAT AAC AGG ATT CCT TCA TAC 8500
Glu Asn Pro Glu Trp Ala Pro Leu Lys Asp Asn Arg Ile Pro Ser Tyr
395 400 405 410
GGG GTC TTG TCT GTT GAT CTG AGT CTG ACA GTT GAG CTT AAA ATC AAA 8548
Gly Val Leu Ser Val Asp Leu Ser Leu Thr Val Glu Leu Lys Ile Lys
415 420 425
ATT GCT TCG GGA TTC GGG CCA TTG ATC ACA CAC GGT TCA GGG ATG GAC 8596
Ile Ala Ser Gly Phe Gly Pro Leu Ile Thr His Gly Ser Gly Met Asp
430 435 440
CTA TAC AAA TCC AAC CAC AAC AAT GTG TAT TGG CTG ACT ATC CCA CCA 8644
Leu Tyr Lys Ser Asn His Asn Asn Val Tyr Trp Leu Thr Ile Pro Pro
445 450 455
ATG AAG AAC CTA GCC TTA GGT GTA ATC AAC ACA TTG GAG TGG ATA CCG 8692
Met Lys Asn Leu Ala Leu Gly Val Ile Asn Thr Leu Glu Trp Ile Pro
460 465 470
AGA TTC AAG GTT AGT CCC TAC CTC TTC AAT GTC CCA ATT AAG GAA GCA 8740
Arg Phe Lys Val Ser Pro Tyr Leu Phe Asn Val Pro Ile Lys Glu Ala
475 480 485 490
GGC GAA GAC TGC CAT GCC CCA ACA TAC CTA CCT GCG GAG GTG GAT GGT 8788
Gly Glu Asp Cys His Ala Pro Thr Tyr Leu Pro Ala Glu Val Asp Gly
495 500 505
GAT GTC AAA CTC AGT TCC AAT CTG GTG ATT CTA CCT GGT CAA GAT CTC 8836
Asp Val Lys Leu Ser Ser Asn Leu Val Ile Leu Pro Gly Gln Asp Leu
510 515 520
CAA TAT GTT TTG GCA ACC TAC GAT ACT TCC AGG GTT GAA CAT GCT GTG 8884
Gln Tyr Val Leu Ala Thr Tyr Asp Thr Ser Arg Val Glu His Ala Val
525 530 535

GTT TAT TAC GTT TAC AGC CCA AGC CGC TCA TTT TCT TAC TTT TAT CCT 8932
Val Tyr Tyr Val Tyr Şer Pro Ser Arg Ser Phe Ser Tyr Phe Tyr Pro
540 545 550
TTT AGG TTG CCT ATA AAG GGG GTC CCC ATC GAA TTA CAA GTG GAA TGC 8980
Phe Arg Leu Pro Ile Lys Gly Val Pro Ile Glu Leu Gln Val Glu Cys
555 560 565 570
TTC ACA TGG GAC CAA AAA CTC TGG TGC CGT CAC TTC TGT GTG CTT GCG 9028
Phe Thr Trp Asp Gln Lys Leu Trp Cys Arg His Phe Cys Val Leu Ala
575 580 585
GAC TCA GAA TCT GGT GGA CAT ATC ACT CAC TCT GGG ATG GTG GGC ATG 9076
Asp Ser Glu Ser Gly Gly His Ile Thr His Ser Gly Met Val Gly Met
590 595 600
GGA GTC AGC TGC ACA GTC ACC CGG GAA GAT GGA ACC AAT CGC AGA 9121
Gly Val Ser Cys Thr Val Thr Arg Glu Asp Gly Thr Asn Arg Arg
605 610 615
TAGGGCTGCT AGTGAACTAA TCTCATGATG TCACCCAGAC ATCAGGCATA CCCACTAGTG 9181
TGAAATAGAC ATCAGAATTA AGAAAAACGT AGGGTCCAAG TGGTTCCCCG TT ATG GAC 9239
Met Asp
TCG CTA TCT GTC AAC CAG ATC TTA TAC CCT GAA GTT CAC CTA GAT AGC 9287
Ser Leu Ser Val Asn Gln Ile Leu Tyr Pro Glu Val His Leu Asp Ser
5 10 15
CCG ATA GTT ACC AAT AAG ATA GTA GCC ATC CTG GAG TAT GCT CGA GTT 9335
Pro Ile Val Thr Asn Lys Ile Val Ala Ile Leu Glu Tyr Ala Arg Val
20 25 30
CCT CAC GCT TAC AGC CTG GAG GAC CCT ACA CTG TGT CAG AAC ATC AAG 9383
Pro His Ala Tyr Ser Leu Glu Asp Pro Thr Leu Cys Gln Asn Ile Lys
35 40 45 50

CAC CGC CTA AAA AAC GGA TTT TCC AAC CAA ATG ATT ATA AAC AAT GTG 9431
His Arg Leu Lys Asn Gly Phe Ser Asn Gln Met Ile Ile Asn Asn Val
55 60 65
GAA GTT GGG AAT GTC ATC AAG TCC AAG CTT AGG AGT TAT CCG GCC CAC 9479
Glu Val Gly Asn Val Ile Lys Ser Lys Leu Arg Ser Tyr Pro Ala His
70 75 80
TCT CAT ATT CCA TAT CCA AAT TGT AAT CAG GAT TTA TTT AAC ATA GAA 9527
Ser His Ile Pro Tyr Pro Asn Cys Asn Gln Asp Leu Phe Asn Ile Glu
85 90 95
GAC AAA GAG TCA ACG AGG AAG ATC CGT GAA CTC CTC AAA AAG GGG AAT 9575
Asp Lys Glu Ser Thr Arg Lys Ile Arg Glu Leu Leu Lys Lys Gly Asn
100 105 110
TCG CTG TAC TCC AAA GTC AGT GAT AAG GTT TTC CAA TGC TTA AGG GAC 9623
Ser Leu Tyr Ser Lys Val Ser Asp Lys Val Phe Gln Cys Leu Arg Asp
115 120 125 130
ACT AAC TCA CGG CTT GGC CTA GGC TCC GAA TTG AGG GAG GAC ATC AAG 9671
Thr Asn Ser Arg Leu Gly Leu Gly Ser Glu Leu Arg Glu Asp Ile Lys
135 140 145
GAG AAA GTT ATT AAC TTG GGA GTT TAC ATG CAC AGC TCC CAG TGG TTT 9719
Glu Lys Val Ile Asn Leu Gly Val Tyr Met His Ser Ser Gln Trp Phe
150 155 160
GAG CCA TTT CTG TTT TGG TTT ACA GTC AAG ACT GAG ATG AGG TCA GTG 9767
Glu Pro Phe Leu Phe Trp Phe Thr Val Lys Thr Glu Met Arg Ser Val
165 170 175
ATT AAA TCA CAA ACC CAT ACT TGC CAT AGG AGG AGA CAC ACA CCT GTA 9815
Ile Lys Ser Gln Thr His Thr Cys His Arg Arg Arg His Thr Pro Val
180 185 190

TTC TTC ACT GGT AGT TCA GTT GAG TTG CTA ATC TCT CGT GAC CTT GTT 9863
Phe Phe Thr Gly Ser Ser Val Glu Leu Leu Ile Ser Arg Asp Leu Val
195 200 205 210
GCT ATA ATC AGT AAA GAG TCT CAA CAT GTA TAT TAC CTG ACA TTT GAA 9911
Ala Ile Ile Ser Lys Glu Ser Gln His Val Tyr Tyr Leu Thr Phe Glu
215 220 225
CTG GTT TTG ATG TAT TGT GAT GTC ATA GAG GGG AGG TTA ATG ACA GAG 9959
Leu Val Leu Met Tyr Cys Asp Val Ile Glu Gly Arg Leu Met Thr Glu
230 235 240
ACC GCT ATG ACT ATT GAT GCT AGG TAT ACA GAG CTT CTA GGA AGA GTC 10007
Thr Ala Met Thr Ile Asp Ala Arg Tyr Thr Glu Leu Leu Gly Arg Val
245 250 255
AGA TAC ATG TGG AAA CTG ATA GAT GGT TTC TTC CCT GCA CTC GGG AAT 10055
Arg Tyr Met Trp Lys Leu Ile Asp Gly Phe Phe Pro Ala Leu Gly Asn
260 265 270
CCA ACT TAT CAA ATT GTA GCC ATG CTG GAG CCT CTT TCA CTT GCT TAC 10103
Pro Thr Tyr Gln Ile Val Ala Met Leu Glu Pro Leu Ser Leu Ala Tyr
275 280 285 290
CTG CAG CTG AGG GAT ATA ACA GTA GAA CTC AGA GGT GCT TTC CTT AAC 10151
Leu Gln Leu Arg Asp Ile Thr Val Glu Leu Arg Gly Ala Phe Leu Asn
295 300 305
CAC TGC TTT ACT GAA ATA CAT GAT GTT CTT GAC CAA AAC GGG TTT TCT 10199
His Cys Phe Thr Glu Ile His Asp Val Leu Asp Gln Asn Gly Phe Ser
310 315 320
GAT GAA GGT ACT TAT CAT GAG TTA ATT GAA GCT CTA GAT TAC ATT TTC 10247
Asp Glu Gly Thr Tyr His Glu Leu Ile Glu Ala Leu Asp Tyr Ile Phe
325 330 335

ATA ACT GAT GAC ATA CAT CTG ACA GGG GAG ATT TTC TCA TTT TTC AGA 10296
Ile Thr Asp Asp Ile His Leu Thr Gly Glu Ile Phe Ser Phe Phe Arg
340 345 350
AGT TTC GGC CAC CCC AGA CTT GAA GCA GTA ACG GCT GCT GAA AAT GTT 10343
Ser Phe Gly His Pro Arg Leu Glu Ala Val Thr Ala Ala Glu Asn Val
355 360 365 370
AGG AAA TAC ATG AAT CAG CCT AAA GTC ATT GTG TAT GAG ACT CTG ATG 10391
Arg Lys Tyr Met Asn Gln Pro Lys Val Ile Val Tyr Glu Thr Leu Met
375 380 385
AAA GGT CAT GCC ATA TTT TGT GGA ATC ATA ATC AAC GGC TAT CGT GAC 10439
Lys Gly His Ala Ile Phe Cys Gly Ile Ile Ile Asn Gly Tyr Arg Asp
390 395 400
AGG CAC GGA GGC AGT TGG CCA CCG CTG ACC CTC CCC CTG CAT GCT GCA 10487
Arg His Gly Gly Ser Trp Pro Pro Leu Thr Leu Pro Leu His Ala Ala
405 410 415
GAC ACA ATC CGG AAT GCT CAA GCT TCA GGT GAA GGG TTA ACA CAT GAG 10535
Asp Thr Ile Arg Asn Ala Gln Ala Ser Gly Glu Gly Leu Thr His Glu
420 425 430
CAG TGC GTT GAT AAC TGG AAA TCT TTT GCT GGA GTG AAA TTT GGC TGC 10583
Gln Cys Val Asp Asn Trp Lys Ser Phe Ala Gly Val Lys Phe Gly Cys
435 440 445 450
TTT ATG CCT CTT AGC CTG GAT AGT GAT CTG ACA ATG TAC CTA AAG GAC 10631
Phe Met Pro Leu Ser Leu Asp Ser Asp Leu Thr Met Tyr Leu Lys Asp
455 460 465
AAG GCA CTT GCT GCT CTC CAA AGG GAA TGG GAT TCA GTT TAC CCG AAA 10679
Lys Ala Leu Ala Ala Leu Gln Arg Glu Trp Asp Ser Val Tyr Pro Lys
470 475 480

GAG TTC CTG CGT TAC GAC CCT CCC AAG GGA ACC GGG TCA CGG AGG CTT 10727
Glu Phe Leu Arg Tyr Asp Pro Pro Lys Gly Thr Gly Ser Arg Arg Leu
485 490 495
GTA GAT GTT TTC CTT AAT GAT TCG AGC TTT GAC CCA TAT GAT GTG ATA 10775
Val Asp Val Phe Leu Asn Asp Ser Ser Phe Asp Pro Tyr Asp Val Ile
500 505 510
ATG TAT GTT GTA AGT GGA GCT TAC CTC CAT GAC CCT GAG TTC AAC CTG 10823
Met Tyr Val Val Ser Gly Ala Tyr Leu His Asp Pro Glu Phe Asn Leu
515 520 525 530
TCT TAC AGC CTG AAA GAA AAG GAG ATC AAG GAA ACA GGT AGA CTT TTT 10871
Ser Tyr Ser Leu Lys Glu Lys Glu Ile Lys Glu Thr Gly Arg Leu Phe
535 540 545
GCT AAA ATG ACT TAC AAA ATG AGG GCA TGC CAA GTG ATT GCT GAA AAT 10919
Ala Lys Met Thr Tyr Lys Met Arg Ala Cys Gln Val Ile Ala Glu Asn
550 555 560
CTA ATC TCA AAC GGG ATT GGC AAA TAT TTT AAG GAC AAT GGG ATG GCC 10967
Leu Ile Ser Asn Gly Ile Gly Lys Tyr Phe Lys Asp Asn Gly Met Ala
565 570 575
AAG GAT GAG CAC GAT TTG ACT AAG GCA CTC CAC ACT CTA GCT GTC TCA 11015
Lys Asp Glu His Asp Leu Thr Lys Ala Leu His Thr Leu Ala Val Ser
580 585 590
GGA GTC CCC AAA GAT CTC AAA GAA AGT CAC AGG GGG GGG CCA GTC TTA 11063
Gly Val Pro Lys Asp Leu Lys Glu Ser His Arg Gly Gly Pro Val Leu
595 600 605 610
AAA ACC TAC TCC CGA AGC CCA GTC CAC ACA AGT ACC AGG AAC GTG AGA 11111
Lys Thr Tyr Ser Arg Ser Pro Val His Thr Ser Thr Arg Asn Val Arg
615 620 625

GCA GCA AAA GGG TTT ATA GGG TTC CCT CAA GTA ATT CGG CAG GAC CAA 11159
Ala Ala Lys Gly Phe Ile Gly Phe Pro Gln Val Ile Arg Gln Asp Gln
630 635 640
GAC ACT GAT CAT CCG GAG AAT ATG GAA GCT TAC GAG ACA GTC AGT GCA 11207
Asp Thr Asp His Pro Glu Asn Met Glu Ala Tyr Glu Thr Val Ser Ala
645 650 655
TTT ATC ACG ACT GAT CTC AAG AAG TAC TGC CTT AAT TGG AGA TAT GAG 11255
Phe Ile Thr Thr Asp Leu Lys Lys Tyr Cys Leu Asn Trp Arg Tyr Glu
660 665 670
ACC ATC AGC TTG TTT GCA CAG AGG CTA AAT GAG ATT TAC GGA TTG CCC 11303
Thr Ile Ser Leu Phe Ala Gln Arg Leu Asn Glu Ile Tyr Gly Leu Pro
675 680 685 690
TCA TTT TTC CAG TGG CTG CAT AAG AGG CTT GAG ACC TCT GTC CTG TAT 11351
Ser Phe Phe Gln Trp Leu His Lys Arg Leu Glu Thr Ser Val Leu Tyr
695 700 705
GTA AGT GAC CCT CAT TGC CCC CCC GAC CTT GAC GCC CAT ATC CCG TTA 11399
Val Ser Asp Pro His Cys Pro Pro Asp Leu Asp Ala His Ile Pro Leu
710 715 720
TAT AAA GTC CCC AAT GAT CAA ATC TTC ATT AAG TAC CCT ATG GGA GGT 11447
Tyr Lys Val Pro Asn Asp Gln Ile Phe Ile Lys Tyr Pro Met Gly Gly
725 730 735
ATA GAA GGG TAT TGT CAG AAG CTG TGG ACC ATC AGC ACC ATT CCC TAT 11495
Ile Glu Gly Tyr Cys Gln Lys Leu Trp Thr Ile Ser Thr Ile Pro Tyr
740 745 750
CTA TAC CTG GCT GCT TAT GAG AGC GGA GTA AGG ATT GCT TCG TTA GTG 11543
Leu Tyr Leu Ala Ala Tyr Glu Ser Gly Val Arg Ile Ala Ser Leu Val
755 760 765 770

CAA GGG GAC AAT CAG ACC ATA GCC GTA ACA AAA AGG GTA CCC AGC ACA 11591
Gln Gly Asp Asn Gln Thr Ile Ala Val Thr Lys Arg Val Pro Ser Thr
775 780 785
TGG CCC TAC AAC CTT AAG AAA CGG GAA GCT GCT AGA GTA ACT AGA GAT 11639
Trp Pro Tyr Asn Leu Lys Lys Arg Glu Ala Ala Arg Val Thr Arg Asp
790 795 800
TAC TTT GTA ATT CTT AGG CAA AGG CTA CAT GAT ATT GGC CAT CAC CTC 11687
Tyr Phe Val Ile Leu Arg Gln Arg Leu His Asp Ile Gly His His Leu
805 810 815
AAG GCA AAT GAG ACA ATT GTT TCA TCA CAT TTT TTT GTC TAT TCA AAA 11735
Lys Ala Asn Glu Thr Ile Val Ser Ser His Phe Phe Val Tyr Ser Lys
820 825 830
GGA ATA TAT TAT GAT GGG CTA CTT GTG TCC CAA TCA CTC AAG AGC ATC 11783
Gly Ile Tyr Tyr Asp Gly Leu Leu Val Ser Gln Ser Leu Lys Ser Ile
835 840 845 850
GCA AGA TGT GTA TTC TGG TCA GAG ACT ATA GTT GAT GAA ACA AGG GCA 11831
Ala Arg Cys Val Phe Trp Ser Glu Thr Ile Val Asp Glu Thr Arg Ala
855 860 865
GCA TGC AGT AAT ATT GCT ACA ACA ATG GCT AAA AGC ATC GAG AGA GGT 11879
Ala Cys Ser Asn Ile Ala Thr Thr Met Ala Lys Ser Ile Glu Arg Gly
870 875 880
TAT GAC CGT TAC CTT GCA TAT TCC CTG AAC GTC CTA AAA GTG ATA CAG 11927
Tyr Asp Arg Tyr Leu Ala Tyr Ser Leu Asn Val Leu Lys Val Ile Gln
885 890 895
CAA ATT CTG ATC TCT CTT GGC TTC ACA ATC AAT TCA ACC ATG ACC CGG 11975
Gln Ile Leu Ile Ser Leu Gly Phe Thr Ile Asn Ser Thr Met Thr Arg
900 905 910

GAT GTA GTC ATA CCC CTC CTC ACA AAC AAC GAC CTC TTA ATA AGG ATG 12023
Asp Val Val Ile Pro Leu Leu Thr Asn Asn Asp Leu Leu Ile Arg Met
915 920 925 930
GCA CTG TTG CCC GCT CCT ATT GGG GGG ATG AAT TAT CTG AAT ATG AGC 12071
Ala Leu Leu Pro Ala Pro Ile Gly Gly Met Asn Tyr Leu Asn Met Ser
935 940 945
AGG CTG TTT GTC AGA AAC ATC GGT GAT CCA GTA ACA TCA TCA ATT GCT 12119
Arg Leu Phe Val Arg Asn Ile Gly Asp Pro Val Thr Ser Ser Ile Ala
950 955 960
GAT CTC AAG AGA ATG ATT CTC GCC TCA CTA ATG CCT GAA GAG ACC CTC 12167
Asp Leu Lys Arg Met Ile Leu Ala Ser Leu Met Pro Glu Glu Thr Leu
965 970 975
CAT CAA GTA ATG ACA CAA CAA CCG GGG GAC TCT TCA TTC CTA GAC TGG 12215
His Gln Val Met Thr Gln Gln Pro Gly Asp Ser Ser Phe Leu Asp Trp
980 985 990
GCT AGC GAC CCT TAC TCA GCA AAT CTT GTA TGT GTC CAG AGC ATC ACT 12263
Ala Ser Asp Pro Tyr Ser Ala Asn Leu Val Cys Val Gln Ser Ile Thr
995 1000 1005 1010
AGA CTC CTC AAG AAC ATA ACT GCA AGG TTT GTC CTG ATC CAT AGT CCA 12311
Arg Leu Leu Lys Asn Ile Thr Ala Arg Phe Val Leu Ile His Ser Pro
1015 1020 1025
AAC CCA ATG TTA AAA GGA TTA TTC CAT GAT GAC AGT AAA GAA GAG GAC 12359
Asn Pro Met Leu Lys Gly Leu Phe His Asp Asp Ser Lys Glu Glu Asp
1030 1035 1040
GAG GGA CTG GCG GCA TTC CTC ATG GAC AGG CAT ATT ATA GTA CCT AGG 12407
Glu Gly Leu Ala Ala Phe Leu Met Asp Arg His Ile Ile Val Pro Arg
1045 1050 1055

GCA GCT CAT GAA ATC CTG GAT CAT AGT GTC ACA GGG GCA AGA GAG TCT 12455
Ala Ala His Glu Ile Leu Asp His Ser Val Thr Gly Ala Arg Glu Ser
1060 1065 1070
ATT GCA GGC ATG CTG GAT ACC ACA AAA GGC CTG ATT CGA GCC AGC ATG 12503
Ile Ala Gly Met Leu Asp Thr Thr Lys Gly Leu Ile Arg Ala Ser Met
1075 1080 1085 1090
AGG AAG GGG GGG TTA ACC TCT CGA GTG ATA ACC AGA TTG TCC AAT TAT 12551
Arg Lys Gly Gly Leu Thr Ser Arg Val Ile Thr Arg Leu Ser Asn Tyr
1095 1100 1105
GAC TAT GAA CAA TTC AGA GCA GGG ATG GTG CTA TTG ACG GGA AGA AAG 12599
Asp Tyr Glu Gln Phe Arg Ala Gly Met Val Leu Leu Thr Gly Arg Lys
1110 1115 1120
AGA AAT GTC CTC ATT GAC AAA GAG TCA TGT TCA GTG CAG CTG GCG AGA 12647
Arg Asn Val Leu Ile Asp Lys Glu Ser Cys Ser Val Gln Leu Ala Arg
1125 1130 1135
GCT CTA AGA AGC CAT ATG TGG GCG AGG CTA GCT CGA GGA CGG CCT ATT 12695
Ala Leu Arg Ser His Met Trp Ala Arg Leu Ala Arg Gly Arg Pro Ile
1140 1145 1150
TAC GGC CTT GAG GTC CCT GAT GTA CTA GAA TCT ATG CGA GGC CAC CTT 12743
Tyr Gly Leu Glu Val Pro Asp Val Leu Glu Ser Met Arg Gly His Leu
1155 1160 1165 1170
ATT CGG CGT CAT GAG ACA TGT GTC ATC TGC GAG TGT GGA TCA GTC AAC 12791
Ile Arg Arg His Glu Thr Cys Val Ile Cys Glu Cys Gly Ser Val Asn
1175 1180 1185
TAC GGA TGG TTT TTT GTC CCC TCG GGT TGC CAA CTG GAT GAT ATT GAC 12839
Tyr Gly Trp Phe Phe Val Pro Ser Gly Cys Gln Leu Asp Asp Ile Asp
1190 1195 1200

AAG GAA ACA TCA TCC TTG AGA GTC CCA TAT ATT GGT TCT ACC ACT GAT 12887
Lys Glu Thr Ser Ser Leu Arg Val Pro Tyr Ile Gly Ser Thr Thr Asp
1205 1210 1215
GAG AGA ACA GAC ATG AAG CTT GCC TTC GTA AGA GCC CCA AGT CGA TCC 12935
Glu Arg Thr Asp Met Lys Leu Ala Phe Val Arg Ala Pro Ser Arg Ser
1220 1225 1230
TTG CGA TCT GCT GTT AGA ATA GCA ACA GTG TAC TCA TGG GCT TAC GGT 12983
Leu Arg Ser Ala Val Arg Ile Ala Thr Val Tyr Ser Trp Ala Tyr Gly
1235 1240 1245 1250
GAT GAT GAT AGC TCT TGG AAC GAA GCC TGG TTG TTG GCT AGG CAA AGG 13031
Asp Asp Asp Ser Ser Trp Asn Glu Ala Trp Leu Leu Ala Arg Gln Arg
1255 1260 1265
GCC AAT GTG AGC CTG GAG GAG CTA AGG GTG ATC ACT CCC ATC TCA ACT 13079
Ala Asn Val Ser Leu Glu Glu Leu Arg Val Ile Thr Pro Ile Ser Thr
1270 1275 1280
TCG ACT AAT TTA GCG CAT AGG TTG AGG GAT CGT AGC ACT CAA GTG AAA 13127
Ser Thr Asn Leu Ala His Arg Leu Arg Asp Arg Ser Thr Gln Val Lys
1285 1290 1295
TAC TCA GGT ACA TCC CTT GTC CGA GTG GCG AGG TAT ACC ACA ATC TCC 13175
Tyr Ser Gly Thr Ser Leu Val Arg Val Ala Arg Tyr Thr Thr Ile Ser
1300 1305 1310
AAC GAC AAT CTC TCA TTT GTC ATA TCA GAT AAG AAG GTT GAT ACT AAC 13223
Asn Asp Asn Leu Ser Phe Val Ile Ser Asp Lys Lys Val Asp Thr Asn
1315 1320 1325 1330
TTT ATA TAC CAA CAA GGA ATG CTT CTA GGG TTG GGT GTT TTA GAA ACA 13271
Phe Ile Tyr Gln Gln Gly Met Leu Leu Gly Leu Gly Val Leu Glu Thr
1335 1340 1345

TTG TTT CGA CTC GAG AAA GAT ACC GGA TCA TCT AAC ACG GTA TTA CAT 13319
Leu Phe Arg Leu Glu Lys Asp Thr Gly Ser Ser Asn Thr Val Leu His
1350 1355 1360
CTT CAC GTC GAA ACA GAT TGT TGC GTG ATC CCG ATG ATA GAT CAT CCC 13367
Leu His Val Glu Thr Asp Cys Cys Val Ile Pro Met Ile Asp His Pro
1365 1370 1375
AGG ATA CCC AGC TCC CGC AAG CTA GAG CTG AGG GCA GAG CTA TGT ACC 13415
Arg Ile Pro Ser Ser Arg Lys Leu Glu Leu Arg Ala Glu Leu Cys Thr
1380 1385 1390
AAC CCA TTG ATA TAT GAT AAT GCA CCT TTA ATT GAC AGA GAT ACA ACA 13463
Asn Pro Leu Ile Tyr Asp Asn Ala Pro Leu Ile Asp Arg Asp Thr Thr
1395 1400 1405 1410
AGG CTA TAC ACC CAG AGC CAT AGG AGG CAC CTT GTG GAA TTT GTT ACA 13511
Arg Leu Tyr Thr Gln Ser His Arg Arg His Leu Val Glu Phe Val Thr
1415 1420 1425
TGG TCC ACA CCC CAA CTA TAT CAC ATT TTA GCT AAG TCC ACA GCA CTA 13559
Trp Ser Thr Pro Gln Leu Tyr His Ile Leu Ala Lys Ser Thr Ala Leu
1430 1435 1440
TCT ATG ATT GAC CTG GTA ACA AAA TTT GAG AAG GAC CAT ATG AAT GAA 13607
Ser Met Ile Asp Leu Val Thr Lys Phe Glu Lys Asp His Met Asn Glu
1445 1450 1455
ATT TCA GCT CTC ATA GGG GAT GAC GAT ATC AAT AGT TTC ATA ACT GAG 13655
Ile Ser Ala Leu Ile Gly Asp Asp Asp Ile Asn Ser Phe Ile Thr Glu
1460 1465 1470
TTT CTC GTC ATA GAG CCA AGA TTA TTC ACT ATC TAC TTG GGC CAG TGT 13703
Phe Leu Val Ile Glu Pro Arg Leu Phe Thr Ile Tyr Leu Gly Gln Cys
1475 1480 1485 1490

GCG GCC ATC AAT TGG GCA TTT GAT GTA CAT TAT CAT AGA CCA TCA GGG 13751
Ala Ala Ile Asn Trp Ala Phe Asp Val His Tyr His Arg Pro Ser Gly
1495 1500 1505
AAA TAT CAG ATG GGT GAG CTG TTG TCA TCG TTC CTT TCT AGA ATG AGC 13799
Lys Tyr Gln Met Gly Glu Leu Leu Ser Ser Phe Leu Ser Arg Met Ser
1510 1515 1520
AAA GGA GTG TTT AAG GTG CTT GTC AAT GCT CTA AGC CAC CCA AAG ATC 13847
Lys Gly Val Phe Lys Val Leu Val Asn Ala Leu Ser His Pro Lys Ile
1525 1530 1535
TAC AAG AAA TTC TGG CAT TGT GGT ATT ATA GAG CCT ATC CAT GGT CCT 13895
Tyr Lys Lys Phe Trp His Cys Gly Ile Ile Glu Pro Ile His Gly Pro
1540 1545 1550
TCA CTT GAT GCT CAA AAC TTG CAC ACA ACT GTC TGC AAC ATG GTT TAC 13943
Ser Leu Asp Ala Gln Asn Leu His Thr Thr Val Cys Asn Met Val Tyr
1555 1560 1565 1570
ACA TGC TAT ATG ACC TAC CTC GAC CTG TTG TTG AAT GAA GAG TTA GAA 13991
Thr Cys Tyr Met Thr Tyr Leu Asp Leu Leu Leu Asn Glu Glu Leu Glu
1575 1580 1585
GAG TTC ACA TTT CTC TTG TGT GAA AGC GAC GAG GAT GTA GTA CCG GAC 14039
Glu Phe Thr Phe Leu Leu Cys Glu Ser Asp Glu Asp Val Val Pro Asp
1590 1595 1600
AGA TTC GAC AAC ATC CAG GCA AAA CAC TTA TGT GTT CTG GCA GAT TTG 14087
Arg Phe Asp Asn Ile Gln Ala Lys His Leu Cys Val Leu Ala Asp Leu
1605 1610 1615
TAC TGT CAA CCA GGG GCC TGC CCA CCA ATT CGA GGT CTA AGA CCG GTA 14135
Tyr Cys Gln Pro Gly Ala Cys Pro Pro Ile Arg Gly Leu Arg Pro Val
1620 1625 1630

GAG AAA TGT GCA GTT CTA ACC GAC CAT ATC AAG GCA GAG GCT AGG TTA 14183
Glu Lys Cys Ala Val Leu Thr Asp His Ile Lys Ala Glu Ala Arg Leu
1635 1640 1645 1650
TCT CCA GCA GGA TCT TCG TGG AAC ATA AAT CCA ATT ATT GTA GAC CAT 14231
Ser Pro Ala Gly Ser Ser Trp Asn Ile Asn Pro Ile Ile Val Asp His
1655 1660 1665
TAC TCA TGC TCT CTG ACT TAT CTC CGG CGA GGA TCG ATC AAA CAG ATA 14279
Tyr Ser Cys Ser Leu Thr Tyr Leu Arg Arg Gly Ser Ile Lys Gln Ile
1670 1675 1680
AGA TTG AGA GTT GAT CCA GGA TTC ATT TTC GAC GCC CTC GCT GAG GTA 14327
Arg Leu Arg Val Asp Pro Gly Phe Ile Phe Asp Ala Leu Ala Glu Val
1685 1690 1695
AAT GTC AGT CAG CCA AAG ATC GGC AGC AAC AAC ATC TCA AAT ATG AGC 14375
Asn Val Ser Gln Pro Lys Ile Gly Ser Asn Asn Ile Ser Asn Met Ser
1700 1705 1710
ATC AAG GCT TTC AGA CCC CCA CAC GAT GAT GTT GCA AAA TTG CTC AAA 14423
Ile Lys Ala Phe Arg Pro Pro His Asp Asp Val Ala Lys Leu Leu Lys
1715 1720 1725 1730
GAT ATC AAC ACA AGC AAG CAC AAT CTT CCC ATT TCA GGG GGC AAT CTC 14471
Asp Ile Asn Thr Ser Lys His Asn Leu Pro Ile Ser Gly Gly Asn Leu
1735 1740 1745
GCC AAT TAT GAA ATC CAT GCT TTC CGC AGA ATC GGG TTG AAC TCA TCT 14519
Ala Asn Tyr Glu Ile His Ala Phe Arg Arg Ile Gly Leu Asn Ser Ser
1750 1755 1760
G[?][?] TGC [?]AC AAA GCT GTT GAG ATA TCA ACA TTA ATT AGG AGA TGC CTT 14567
[?][?][?] [?]y[?] [?]y[?] [?]y[?] Ala Val Glu Ile Ser Thr Leu Ile Arg Arg Cys Leu
1765 1770 1775

GAG CCA GGG GAG GAC GGC TTG TTC TTG GGT GAG GGA TCG GGT TCT ATG 14615

Glu Pro Gly Glu Asp Gly Leu Phe Leu Gly Glu Gly Ser Gly Ser Met

1780 1785 1790

TTG ATC ACT TAT AAG GAG ATA CTT AAA CTA AAC AAG TGC TTC TAT AAT 14663

Leu Ile Thr Tyr Lys Glu Ile Leu Lys Leu Asn Lys Cys Phe Tyr Asn

1795 1800 1805 1810

AGT GGG GTT TCC GCC AAT TCT AGA TCT GGT CAA AGG GAA TTA GCA CCC 14711

Ser Gly Val Ser Ala Asn Ser Arg Ser Gly Gln Arg Glu Leu Ala Pro

1815 1820 1825

TAT CCC TCC GAA GTT GGC CTT GTC GAA CAC AGA ATG GGA GTA GGT AAT 14759

Tyr Pro Ser Glu Val Gly Leu Val Glu His Arg Met Gly Val Gly Asn

1830 1835 1840

ATT GTC AAA GTG CTC TTT AAC GGG AGG CCC GAA GTC ACG TGG GTA GGC 14807

Ile Val Lys Val Leu Phe Asn Gly Arg Pro Glu Val Thr Trp Val Gly

1845 1850 1855

AGT GTA GAT TGC TTC AAT TTC ATA GTT AGT AAT ATC CCT ACC TCT AGT 14855

Ser Val Asp Cys Phe Asn Phe Ile Val Ser Asn Ile Pro Thr Ser Ser

1860 1865 1870

GTG GGG TTT ATC CAT TCA GAT ATA GAG ACC TTG CCT AAC AAA GAT ACT 14903

Val Gly Phe Ile His Ser Asp Ile Glu Thr Leu Pro Asn Lys Asp Thr

1875 1880 1885 1890

ATA GAG AAG CTA GAG GAA TTG GCA GCC ATC TTA TCG ATG GCT CTG CTC 14951

Ile Glu Lys Leu Glu Glu Leu Ala Ala Ile Leu Ser Met Ala Leu Leu

1895 1900 1905

CTG GGC AAA ATA GGA TCA ATA CTG GTG ATT AAG CTT ATG CCT TTC AGC 14999

Leu Gly Lys Ile Gly Ser Ile Leu Val Ile Lys Leu Met Pro Phe Ser

1910 1915 1920

GGG GAT TTT GTT CAG GGA TTT ATA AGT TAT GTA GGG TCT TAT TAT AGA 15047
Gly Asp Phe Val Gln Gly Phe Ile Ser Tyr Val Gly Ser Tyr Tyr Arg
1925 1930 1935
GAA GTG AAC CTT GTA TAC CCT AGA TAC AGC AAC TTC ATA TCT ACT GAA 15095
Glu Val Asn Leu Val Tyr Pro Arg Tyr Ser Asn Phe Ile Ser Thr Glu
1940 1945 1950
TCT TAT TTG GTT ATG ACA GAT CTC AAG GCT AAC CGG CTA ATG AAT CCT 15143
Ser Tyr Leu Val Met Thr Asp Leu Lys Ala Asn Arg Leu Met Asn Pro
1955 1960 1965 1970
GAA AAG ATT AAG CAG CAG ATA ATT GAA TCA TCT GTG AGG ACT TCA CCT 15191
Glu Lys Ile Lys Gln Gln Ile Ile Glu Ser Ser Val Arg Thr Ser Pro
1975 1980 1985
GGA CTT ATA GGT CAC ATC CTA TCC ATT AAG CAA CTA AGC TGC ATA CAA 15239
Gly Leu Ile Gly His Ile Leu Ser Ile Lys Gln Leu Ser Cys Ile Gln
1990 1995 2000
GCA ATT GTG GGA GAC GTA GTT AGT AGA GGT GAT ATC AAT CCT ACT CTG 15287
Ala Ile Val Gly Asp Val Val Ser Arg Gly Asp Ile Asn Pro Thr Leu
2005 2010 2015
AAA AAA CTT ACA CCT ATA GAG CAG GTG CTG ATC AAT TGC GGG TTG GCA 15335
Lys Lys Leu Thr Pro Ile Glu Gln Val Leu Ile Asn Cys Gly Leu Ala
2020 2025 2030
ATT AAC GGA CCT AAG CTG TGC AAA GAA TTG ATC CAC CAT GAT GTT GCC 15383
Ile Asn Gly Pro Lys Leu Cys Lys Glu Leu Ile His His Asp Val Ala
2035 2040 2045 2050
TCA GGG CAA GAT GGA TTG CTT AAT TCT ATC CTC ATC CTC TAC AGG GAG 15431
Ser Gly Gln Asp Gly Leu Leu Asn Ser Ile Leu Ile Leu Tyr Arg Glu
2055 2060 2065

TTG GCA AGA TTC AAA GAC AAC CGA AGA AGT CAA CAA GGG ATG TTC CAC 15479
Leu Ala Arg Phe Lys Asp Asn Arg Arg Ser Gln Gln Gly Met Phe His
2070 2075 2080
GCT TAC CCC GTA TTG GTA AGT AGC AGG CAA CGA GAA CTT ATA TCT AGG 15527
Ala Tyr Pro Val Leu Val Ser Ser Arg Gln Arg Glu Leu Ile Ser Arg
2085 2090 2095
ATC ACC CGC AAA TTT TGG GGG CAC ATT CTT CTT TAC TCC GGG AAC AGA 15575
Ile Thr Arg Lys Phe Trp Gly His Ile Leu Leu Tyr Ser Gly Asn Arg
2100 2105 2110
AAG TTG ATA AAT AAG TTT ATC CAG AAT CTC AAG TCC GGC TAT CTG ATA 15623
Lys Leu Ile Asn Lys Phe Ile Gln Asn Leu Lys Ser Gly Tyr Leu Ile
2115 2120 2125 2130
CTA GAC TTA CAC CAG AAT ATC TTC GTT AAG AAT CTA TCC AAG TCA GAG 15671
Leu Asp Leu His Gln Asn Ile Phe Val Lys Asn Leu Ser Lys Ser Glu
2135 2140 2145
AAA CAG ATT ATT ATG ACG GGG GGT TTG AAA CGT GAG TGG GTT TTT AAG 15719
Lys Gln Ile Ile Met Thr Gly Gly Leu Lys Arg Glu Trp Val Phe Lys
2150 2155 2160
GTA ACA GTC AAG GAG ACC AAA GAA TGG TAT AAG TTA GTC GGA TAC AGT 15767
Val Thr Val Lys Glu Thr Lys Glu Trp Tyr Lys Leu Val Gly Tyr Ser
2165 2170 2175
GCC CTG ATT AAG GAC TAATTGGTTG AACTCCGGAA CCCTAATCCT GCCCTAGGTG 15822
Ala Leu Ile Lys Asp
2180
GTTAGGCATT ATTTGCAATA TATTAAAGAA AACTTTGAAA ATACGAAGTT TCTATTCCCA 15882
GCTTTGTCTG GT 15894

Sequence list of the above nucleotide sequence.
- Sequence type: : nucleic acid
- Strandedness: : single
- Topology: : linear
- Molecule type: : cDNA

### Original source

- Organism: : attenuated measles vaccine
- Strain: : AIK-C

Accordingly, the present invention provides a method for identifying a measles vaccine virus strain, which method comprises determining the nucleotide sequence of cDNA corresponding to the genomic RNA of the measles vaccine virus strain and comparing the sequence thus determined with the DNA sequence set out in Sequence I.D. No. 1.

The measles virus vaccine strain may have been taken from an infant vaccinated with the AIK-C strain of measles virus. In an alternative embodiment, measles virus for vaccines is produced from seed AIK-C strain measles virus and the quality of the virus thus produced is checked by determining the nucleotide sequence of the cDNA corresponding to the genomic RNA of the virus and comparing the sequence thus determined with the DNA sequence set out in Sequence I.D. No. 1.

The invention also provides a cDNA, for example an isolated cDNA, having the nucleotide sequence set out in Sequence I.D. No. 1. The invention further provides a RNA having a nucleotide sequence corresponding to that set out in the Sequence I.D. No. 1.

### In the accompanying drawings:

Fig. 1 is a outline of cDNA construction.

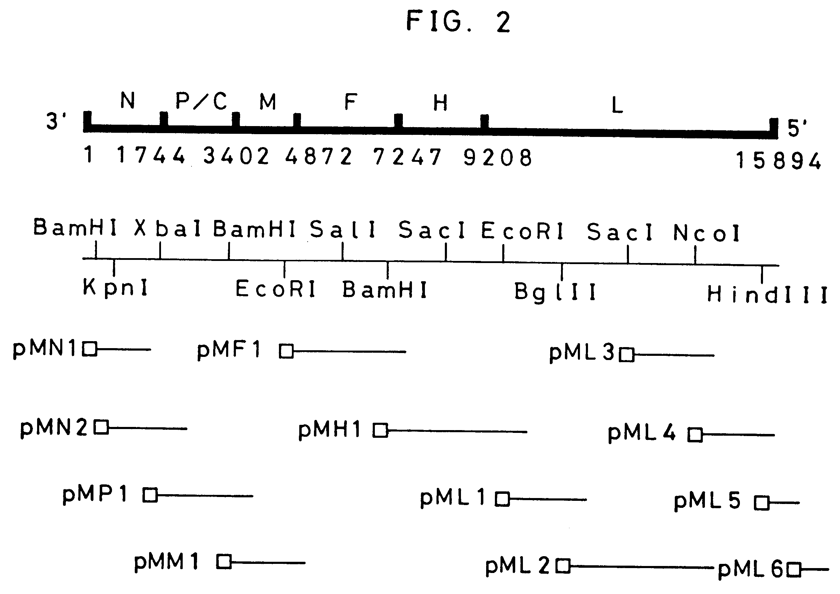

Fig. 2 is a map of cDNA clones of the AIK-C, and
Fig. 3 sets out primer sequences.

In the present invention, at first, seed virus of the measles virus AIK-C strain is used. This virus is propagated in chick embryo cells derived from specific pathogen-free hen's eggs to prepare so-called vaccine bulk, from which a virus suspension can be prepared for purification. The vaccine bulk can be a part of a virus pool, prepared for the market, of measles AIK-C strain vaccine.

The clarified virus suspension is purified by ultra-centrifugation through continuous sucrose gradients. Virus RNA is extracted by SDS-phenol method. Synthetic primer can be synthesized with approx. 25 mer synthetic primer by amidide method.

Deoxyoligonucleotides, used as synthetic primers, can be synthesized, for example, on a CYCLONE DNA SYNTHESIZER (Biosearch, Inc.).

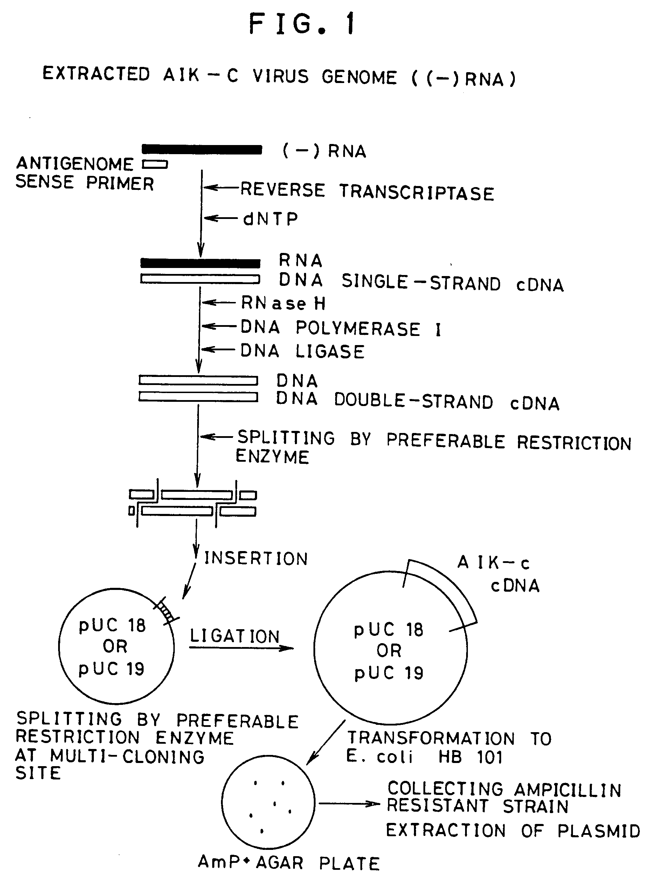

The AIK-C virus genome RNA is used as a template for the synthesis of cDNA using the above synthetic oligonucleotide primer. The AIK-C virus genome RNA is reverse-transcribed by reverse transcriptase to prepare single-strand cDNA which is treated by the RNase H method to prepare double-strand cDNA. The double-stranded cDNAs are treated with appropriate restriction enzymes and are inserted into the pUC plasmid ( pUC 18 or pUC 19). The above process is illustrated in Fig. 1. E. coli K 12 strain HB101 is transformed with the resulting recombinant plasmid. The transformants are selected by ampicillin resistance. Colonies containing recombinant plasmids are screened by measuring the size of palsmid DNA with agarose gel electrophoresis. To determine the AIK-C genome sequence, the cDNA is subcloned into the bacteriophage M 13 series, and the single-strand M 13 phage DNAs are isolated from candidate subclones.

Determination of the obtained cDNA clone can be made by several restriction enzyme cleavage types. In the cDNA obtained from synthetic primer such as MP-1, restriction enzyme sites of BamHI, EcoRV and XbaI are located. In the plasmid extracted from ampicillin resistant strain, in case that the said type of cDNA is observed, approximately 1504 base fragments are identified by splitting with BamHI and XbaI on an agarose gel electrophoresis. Further treatment of these fragments with EcoRV provides two fragments of 650 and 854 base fragments. Accordingly, an identification of the obtained cDNA clone has been made by cleaving the inserted fragment in the plasmid and its size determination, or by determining the specific restriction site of measles DNA in the fragments.

To determine the base sequence, the cDNA, which is split by a restriction enzyme to prepare cohesive or blunt ends, is inserted into M 13 phage vector in which its cloning parts are cleaved by the preferable restriction enzymes. The phage holding the thus prepared recombinant DNA is infected into E. coli. Single-strand recombinant DNA is extracted from the infected phage plaques, from which the nucleotide sequence is determined by the dideoxy chain termination method ( dideoxy sequence method ).

A primer such as pMN 2, the cDNA obtained from MP-1, is split by BamHI and XbaI, and is cloned into a pUC plasmid. After determining cDNA, the DNA fragment originating from the AIK-C strain by splitting with the same restriction enzyme is further split by EcoRV to prepare two fragments consisting of 650 bp and 854 bp.

These fragments are ligated into M 13 replicative form double-strand DNA, which had previously been split by BamHI and XbaI, and transfected into E. coli. After recombinant phage is selected, the phage is infected in E. coli and multiplied. Single-strand recombinant DNA is extracted from supernatant phase of phage solution and nucleotide sequence is determined by the dideoxy sequence method.

The virus genome of AIK-C strain has been found to consist of 15,894 nucleotides and its whole sequence is shown above. A measles virus containing the said RNA genome has the superior properties of the AIK-C virus strain. The specific nucleotide sequence of AIK-C virus strain genome is determined by PCR and can be compared with the virus strain to determine whether the said strain can be used for AIK-C strain virus vaccine or whether the prepared AIK-C strain virus vaccine is strictly holding the properties of AIK-C strain. Further, if an infant to whom measles vaccine has been administered shows exanthem and measles virus antibody is observed in lymphocytes of the infant, the cause can be determined by comparing the nucleotide sequence of the measles vaccine virus with the nucleotide sequence of the AIK-C virus.

In the specification, following abbreviations are used.
- Met ;: methionine
- Ala ;: alanine
- Thr ;: threonine
- Leu ;: leucine
- Arg ;: arginine
- Ser ;: serine
- Phe ;: phenylalanine
- Lys ;: lysine
- Asn ;: asparagine
- Asp ;: aspartic acid
- Pro ;: proline
- Ile ;: isoleucine
- Gly ;: glycine
- His ;: histidine
- Val ;: valine
- Glu ;: glutamic acid
- Gln ;: glutamine

Following examples illustrate the present invention but are not construed as limiting.

### Example 1

### Determination of Nucleotide sequence of a virus obtained from AIK-C virus strain vaccine bulk:

### Step : a

A seed virus of measles AIK-C strain ( Seed Lot 0 - 2 ) was inoculated up to an infectivity titer of 0.05 in chick embryo cells derived from specific pathogen-free hen's egg cultured in a surface area of cell culture ( 230 cm² ) in a large Roux bottle ( approx. 1 liter volume). A culture medium ( 150 ml ) of Eagle's minimum essential medium ( MEM ) containing 1 % calf serum was added therein and the virus was cultured at 33 °C.

### Step : b

On the third day after inoculation, cultured medium was removed, then new medium ( 150 ml ), the same as above, was further added and the cultivation was continued.

### Step : c

After inoculation of virus, on the sixth day, cultured medium was removed. Infected chick embryo cells layer was washed three times with Hank's solution ( 100 ml ) completely. Further cultivation was continued at 33°C after adding Eagle's MEM for cell culture ( 200 ml ) containing 0.1 % sodium glutamate.

### Step : d

On the tenth day after virus seed inoculation wherein specific cytopathogenic effect for measles virus was observed on whole infected cell layer, the culture medium was collected for vaccine bulk solution ( volume : approx. 200 ml, infectivity titer : 10^{7.2} TCID₅₀ * /ml ) ( * medium tissue culture infective dose ).

### Step : e

Vaccine bulk solution was centrifuged at 3,000 r.p.m. for 30 minutes and the supernatant solution was further centrifuged at 25,000 r.p.m. for 90 minutes. ( Beckman. L 8 - 55 M, rotor : SW 28)

### Step : f

After supernatant solution was removed, TEN buffer solution (10 mM-Tris HCl, 1 mM-EDTA, 100 mM-NaCl, pH 7.4 )( 1 ml ) was added to a precipitate in each centrifugal tube, and suspended well to prepare concentrated virus. The thus obtained concentrated virus suspension was adjusted to a final volume of 10 ml by addition of TEN buffer solution to prepare a clarified virus suspension.

### Step : g

The clarified virus suspension ( 5 ml ) was layered on the 30 ∼ 60 % continuous sucrose gradients in two centrifugal tubes ( Beckman centrifuge SW 41 rotor ), prepared by 60 % ( w/v ) sucrose solution in TEN buffer solution ( 6.8 ml ) and 30 % ( w/v ) sucrose solution in TEN buffer solution (6.8 ml ) per tube, then centrifuged for 90 minutes at 207,000 g at 4 °C. The suspension was fractionated and the fractions showing highest infectivity titer ( 10^{8.3} TCID₅₀/ml ) were collected and again centrifuged by the same way to prepare purified virus particles.

### Step : h

The purified virus particles were diluted fivefold with TEN buffer solution, separately added each 200µl into 1.5 ml microtest tube, further added 20 % SDS ( 5 µl, phenol ( 100 µl ) and chloroform ( 100µl ) therein, then stirred completely using a vortex mixer. The mixture was further centrifuged at 12,000 r.p.m. for 5 minutes to separate the organic layer from the aqueous layer. The aqeous layer was collected into another 1.5 ml microtest tube and subjected twice with phenol extraction ( SDS-phenol extraction ). The recovered aqueous layer was mixed with 5M-NaCl (1/25 volume ), and ethanol (2.5 volume ), allowed to stand at -20°C for 2 hours, then centrifuged at 12,000 r.p.m. for 10 minutes to collect precipitated RNA which was washed with 70 % ethanol and dried. The dried material was dissolved in double-distilled water( 50 µl )which was sterilized by autoclaving to prepare a RNA suspension.

### Step : i

### Cyclone DNA synthesizer

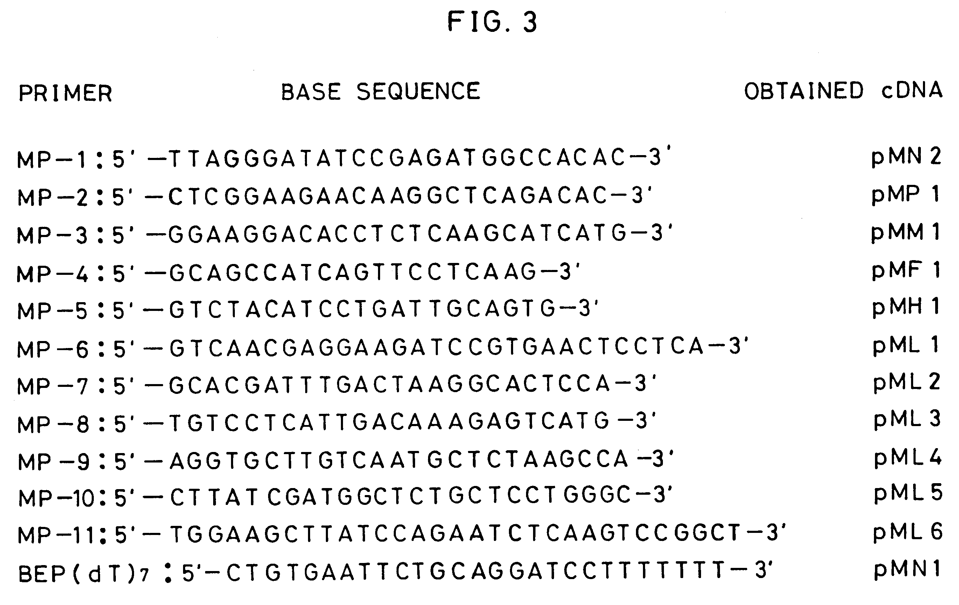

Synthetic primer deoxyoligonucleotides, in particular the 12 primers comprising approx. 25 mer of MP-1 ∼ MP-11 and BEP( dT )₇ shown in Fig. 3, were synthesized by using Cyclone DNA synthesizer ( Biosearch INC. U.S.A. ).

### Step : j

The RNA suspension ( 10µl ) obtained in the above Step : h ( AIK-C virus genome RNA ) was used as template for the synthesis of DNA using synthetic oligonucleotide primers ( the above synthetic DNA )( 2 µl of synthetic primer, MP-1 or MP-11 ) and cDNA was prepared by reverse transcriptase treatment. The cDNA was converted to double-strand cDNA by using RNase H-DNA polymerase I ( Gubler and Hoffman, GENE 25, p 263 - 269, 1983 ).

### Step : k

The obtained cDNA was cleaved at each restriction enzyme cleavage site by BamHI-XbaI, XbaI-BamHI, BamHI-EcoRI, EcoRI-BamHI, BamHI-EcoRI, EcoRI-BglII, BglII-SacI and SacI-NcoI-XbaI. Each fragment was inserted into a corresponding cloning site in pUC plasmid ( pUC 18 and pUC 19 ).

### Step : l

E. coli HB101 was transformed with the above recombinant plasmids to obtain ampicillin resistant colonies. Plasmid DNA was extracted from the thus obtained colonies and the colonies containing recombinant plasmids were screened by measuring the size length of the fragments plasmid DNA with 0.8 % agarose gel electrophoresis.

### Step : m

To obtain the 3' terminal clone of the AIK-C genome, poly ( A ) was tailed at the 3' end of the genomic RNA, an RNA suspension obtained in the above step : h, with poly ( A ) polymerase and adenosine triphosphate ( ATP ). The thus obtained 3' A tailed RNA suspension, i. e. the polyadenylated RNA was reversely transcribed using BEP ( dT )₇ primer to prepare cDNA according to the step : j. The BEP ( dt )₇ primer has the sequence ; 5'-CTGTGAATTCTGCAGGATCCTTTTTTT-3'.

### Step : n

The 5' terminal clone was synthesized using a primer located close to the 5' terminus. Namely the primer contained complementary DNA in the domain of nucleotides 15,592-15,615 of the measles virus genome. The synthetic primer has the sequence ; 5'-TGGAAGCTTATCCAGAATCTCAAGTCCGGCT-3'.

DNA-RNA hybrid was prepared by using the said synthetic DNA as a primer with reverse transcriptase. After alkaline treatment of the hybrid, poly ( dA ), i. e. dATP was tailed to the 3' end of the resulting cDNA with terminal deoxynucleotidyl transferase. It was subsequently converted to the double-stranded cDNA using the BEP ( dT )₇ primer in the step : m and the Klenow fragment.

### Step : o

The thus obtained cDNAs were subcloned into the bacteriophage M 13 series vector ( mp 18 and mp 19 ), and the single-strand M 13 Phage DNAs were isolated. Nucleotide sequence of those cDNAs was determined with the said single-stranded DNA by means of the dideoxy chain termination method using 7-DEAZA-dGTP sequencing Kit ( Takara Shuzo ).

### Step : p

Computer analysis of the nucleotide and peptide sequence were performed by GENETYX software.

### Example 2

### Determination of viral nucleotide of AIK-C strain seed virus grown in Vero cells ( African green monkey cell live )

### Step : a

AIK-C strain seed virus ( Seed Lot No. 0 - 2 ) was inoculated into Vero cells previously cultured in a large size Roux bottle according to the method described in the Example 1, and incubated at 33 °C.

### Step : b

On the day 5th of incubation, infected cell layers were washed with Hank's solution, then Eagle's MEM ( 200 ml ) without calf serum was added thereto and further incubated for 2 days. Incubated viral culture was collected to obtain virus bulk suspension ( approx. 200 ml, infective titer : 10^{6.5} TCID₅₀/ml ).

### Step : c

The vaccine bulk was centrifuged at 3,000 r.p.m. for 30 min., thereafter the supernatant suspension was centrifuged at 25,000 r.p.m. for 90 min. [ Centrifuge: Beckman L8-55M, rotor : SW 28]

### Step : d

After the supernatant solution was removed, TEN buffer solution (10 mM Tris-HCl, 1 mM EDTA, 100 mM NaCl, pH 7.4, 1 ml ) was added to the precipitate in each centrifugal tube. The precipitate was suspended completely to prepare conc. virus material, which was adjusted to a volume of 10 ml by adding TEN buffer solution to prepare a starting virus suspension.

### Step : e

The starting virus suspension ( 5 ml ) was layered on a 30 - 60 % continuous sucrose gradients in two tubes consisting of, in one tube, 60 % ( w/v ) sucrose solution ( TEN buffer solution )( 6.8 ml ) and 30 % ( w/v ) sucrose solution ( TEN buffer solution )( 6.8 ml ), then centrifuged at 207,000 g for 90 min. at 4 °C.

The centrifuged suspension in each tube was fractionated by means of a fraction collector and fractions showing high infectivity ( 10^{8.3} TCID₅₀/ml ) were collected. The collected fraction was again centrifuged by the same manner to recover purified virus.

### Step : f

200 µl aliquots of purified virus diluted fivefold with TEN buffer solution were separately introduced into 1.5 ml micro test tubes. Thereto was added 20 % SDS ( 5 µl ), phenol ( 100µl ) and chloroform ( 100µl ), and completely stirred using a vortex mixer. The organic and aqueous layers were separated by a centrifuge at 12,000 r.p.m. for 5 min. The aqueous layer was collected in another 1.5 ml micro test tube, and extracted twice with the above same SDS-phenol.

Recovered aqueous layer was mixed with 5 M NaCl ( 1/25 volume ) and ethanol ( 2.5 volume ), allowed to stand at -20°C for 2 hours, centrifuged at 12,000 r.p.m. for 10 min. to collect precipitated RNA, which was washed with 70 % ethanol and dried. RNA suspension was prepared with sterilisation of the dried material and dissolved in redistilled water ( 50 µl ).

### Step : g

### Cyclone DNA synthesizer

Synthetic primer deoxyoligonucleotides, in particular the 12 primers, comprising approx. 25 mer of MP-1∼ MP-11 and BEP ( dT )₇ shown in Fig. 3, were synthesized by using cyclone DNA synthesizer ( Biosearch Inc. U.S.A. ).

### Step : h

The RNA suspension ( 10 µl ) obtained in the above Example : 1 ( AIK-C virus genome RNA ) was used as a template for the synthesis of cDNA using the synthetic oligonucleotide primers ( the above synthetic DNA ) ( 2µl of synthetic primer, MP-1 or MP-11 ) and cDNA was prepared by reverse transcriptase treatment. The cDNA was converted to double-strand cDNA by using RNaseH-DNA polymerase I ( Gubler and Hoffman, GENE 25, p. 263-269, 1983).

### Step : i

The obtained cDNA was cleaved at each restriction enzyme cleavage site by BamHI-XbaI, XbaI-BamHI, BamHI-EcoRI, EcoRI-BamHI, BamHI-EcoRI, EcoRI-Bg1II, Bg1II-SacI and SacI-NcoI-XbaI. Each fragment was inserted into a corresponding cloning site in pUC plasmid ( pUC18 and pUC 19 ).

### Step : j

E. coli HB101 was transformed with the above recombinant plasmids to obtain ampicillin resistant colonies. Plasmid DNA was extracted from the thus obtained colonies and the colonies containing recombinant plasmids were screened by measuring the size length of fragment plasmid DNA with 0.8 % agarose gel electrophoresis.

### Step : k

To obtain the 3' terminal clone of the AIK-C genome, poly ( A ) was tailed at the 3' end of the genomic RNA, an RNA suspension obtained in the above step : f, with poly ( A ) polymerase and adenosine triphosphate ( ATP ). The thus obtained 3' tailed RNA suspension, i. e. the polyadenylated RNA was reversely transcribed using BEP ( dT )₇ primer to prepare cDNA according to the step : h. The BEP ( dT )₇ primer has the sequence ; 5'-CTGTGAATTCTGCAGGATCCTTTTTTT-3'.

### Step : l

The 5' terminal clone was synthesized using a primer located close to the 5' terminus. Namely the primer contained complementary DNA in the domain of nucleotides 15,592-15,615 of the measles virus genome. The synthetic primer has the sequence ; 5'-TGGAAGCTTATCCAGAATCTCAAGTCCGGCT-3'.

DNA-RNA hybrid was prepared by using the said synthetic DNA as a primer with reverse transcriptase. After alkaline treatment of the hybrid, poly ( dA ), i. e. dATP was tailed to the 3' end of the resulting cDNA with terminal deoxynucleotidyl transferase. It was subsequently converted to the double-stranded cDNA using the BEP ( dT )₇ primer in the step : k and the Klenow fragment.

### Step : m

The thus obtained cDNAs were subcloned into the bacteriophage M 13 series vector ( mp 18 and mp 19 ), and the single-stranded M 13 phage DNAs were isolated. Nucleotide sequence of those cDNAs was determined with the said single-stranded DNA by means of the dideoxy chain termination method using 7-DEAZA-dGTP sequencing kit ( Takara Shuzo ).

### Step : n

Computer analysis of the nucleotide and peptide sequences were performed by GENETYX software.

### Example 3

### Identification by PCR of measles virus obtained from a patient on the first day that a measles infection appeared

### Step : a

A blood specimen ( approx. 5 ml ) was collected from a patient on the first day that measles exanthem appeared. Lymphocytes were separately by Ficoll ( trade name).

### Step : b

The lymphocytes were washed twice with PBS. A denaturation solution D ( 4M guanidium thiocyanate, 25 mM sodium citrate, pH 7.5 ; 0.5 % sacrosine, 0.1 M 2-mercapto ethanol, 200 µl ) was added thereto. After gently stirred, 2 M sodium acetate ( 20µl, pH 4 ), phenol ( 200 µl ) and chloroform ( 100 µl ) were added in this order.

The mixture was treated with vortex mixer for 10 sec. and allowed to stand in ice-water for 15 min. Then the mixture was centrifuged at 10,000 g for 20 min. to recover the aqueous layer. An equal volume of isopropanol was added. The mixture was allowed to stand at -20 °C for one hour and then centrifuged at 10,000 g for 20 min. to precipitate RNA [ Chomczynski and Sacchi, Anal. Biochem., 162, 156-159 ( 1987 ) ].

### Step : c

The thus obtained RNA was subjected to the reverse transcriptase reaction and PCR method.

### Step : d

Nucleotide sequencing and identification of measles virus AIK-C vaccine strain and wild strain were performed.

## Claims

1. A method for identifying a measles vaccine virus strain, which method comprises determining the nucleotide sequence of cDNA corresponding to the genomic RNA of the measles vaccine virus strain and comparing the sequence thus determined with the DNA sequence set out in Sequence I.D. No. 1.

2. A method according to claim 1, wherein the measles virus vaccine strain has been taken from an infant vaccinated with the AIK-C strain of measles virus.

3. A method according to claim 1, wherein measles virus for vaccines is produced from seed AIK-C strain measles virus and the quality of the virus thus produced is checked by determining the nucleotide sequence of cDNA corresponding to the genomic RNA of the virus and comparing the sequence thus determined with the DNA sequence set out in Sequence I.D. No. 1.

4. A cDNA having the nucleotide sequence set out in Sequence I.D. No.1.

5. An isolated cDNA according to claim 4.

6. A RNA having a nucleotide sequence corresponding to that set out in Sequence I.D. No. 1.

## Patentansprüche

1. Verfahren zur Identifizierung eines Masernvakzine-Virusstamms, das umfaßt: Bestimmen der Nucleotidsequenz der der genomischen RNA des Masern-Virusstammes entsprechenden cDNA und Vergleichen der so bestimmten Sequenz mit der in Sequenz I.D. No. 1 angegebenen DNA-Sequenz.

2. Verfahren nach Anspruch 1, dadurch gekennzeichnet, daß der Masernvakzine-Virusstamm einem mit dem AIK-C-Stamm von Masernvirus geimpftem Kind entnommen wurde.

3. Verfahren nach Anspruch 1, dadurch gekennzeichnet, daß der Masernvirus für Vakzine aus Impfkultur-AIK-C-Stamm-Masernvirus hergestellt wird und die Qualität des so hergestellten Virus durch Bestimmen der Nucleotidsequenz der der genomischen RNA des Virus entsprechenden cDNA geprüft wird, und die so bestimmte Sequenz mit der in Sequenz I.D. No. 1 angegebenen DNA-Sequenz verglichen wird.

4. cDNA mit der in Sequenz I.D. No. 1 angegebenen Nucleotidsequenz.

5. Isolierte cDNA nach Anspruch 4.

6. RNA mit einer Nucleotidsequenz, die der in Sequenz I.D. No. 1 angegebenen entspricht.

## Revendications

1. Procédé d'identification d'une souche de vaccin viral antirougeoleux, ledit procédé comprenant la détermination de la séquence nucléotidique d'ADNc correspondant à l'ARN génomique de la souche de vaccin viral antirougeoleux et la comparaison de la séquence ainsi déterminée à la séquence d'ADN indiquée dans la Séquence I.D. n° 1.

2. Procédé selon la revendication 1, dans lequel la souche de vaccin viral antirougeoleux est prélevée sur un nourrisson vacciné avec la souche AIK-C du virus de la rougeole.

3. Procédé selon la revendication 1, dans lequel le virus de la rougeole pour vaccins est produit à partir de germes du virus de la rougeole de souche AIK-C, et on vérifie la qualité du virus ainsi produit en déterminant la séquence nucléotidique d'ADNc correspondant à l'ARN génomique du virus et en comparant la séquence ainsi déterminée à la séquence d'ADN indiquée dans la Séquence I.D. n° 1.

4. ADNc ayant la séquence nucléotidique indiquée dans la Séquence I.D. n° 1.

5. ADNc isolé selon la revendication 4.

6. ARN ayant une séquence nucléotidique correspondant à celle indiquée dans la Séquence I.D. n° 1.
